# EUROPÄISCHE PATENTSCHRIFT

(11) **EP 1 769 080 B1**
(45) Veröffentlichungstag und Bekanntmachung des Hinweises auf die Patenterteilung: **04.09.2013**
(21) Anmeldenummer: 05773106.9
(22) Anmeldetag: 20.07.2005
(51) Int. Cl.: C12P 13/12

(54) **MIKROORGANISMEN ZUR HERSTELLUNG VON SCHWEFELHALTIGEN VERBINDUNGEN**
MICROORGANISMS FOR PRODUCING SULPHUR-CONTAINING COMPOUNDS
MICRO-ORGANISMES POUR LA PRODUCTION DE COMPOSES SULFURES

(30) Priorität: 20.07.2004 DE 102004035052
(43) Veröffentlichungstag der Anmeldung: 04.04.2007
(73) Patentinhaber: Evonik Degussa GmbH, 45128 Essen (DE)
(72) Erfinder: SAUER, Uwe, CH-8049 Zürich (CH); MAMPEL, Jörg, 64625 Bensheim (DE); SCHRÖDER, Hartwig, 69226 Nussloch (DE); HAEFNER, Stefan, 67346 Speyer (DE); ZELDER, Oskar, 67346 Speyer (DE); HEROLD, Andrea, 68775 Ketsch (DE); KLOPPROGGE, Corinna, 68239 Mannheim (DE)
(86) Internationale Anmeldenummer: PCT/EP2005/007925
(87) Internationale Veröffentlichungsnummer: WO 2006/008152

(56) Entgegenhaltungen:
- WO-A-03/023044
- WO-A-2004/050694
- US-A1- 2003 162 267
- REY D A ET AL: "THE PUTATIVE TRANSCRIPTIONAL REPRESSOR MCBR, MEMBER OF THE TETR-FAMILY, IS INVOLVED IN THE REGULATION OF THE METABOLIC NETWORK DIRECTING THE SYNTHESIS OF SULFUR CONTAINING AMINO ACIDS IN CORYNEBACTERIUM GLUTAMICUM" JOURNAL OF BIOTECHNOLOGY, ELSEVIER SCIENCE PUBLISHERS B.V., AMSTERDAM, NL, Bd. 103, Nr. 1, 12. Juni 2003 (2003-06-12), Seiten 51-65, XP001152914 ISSN: 0168-1656
- RÜCKERT C ET AL: "Genome-wide analysis of the L-methionine biosynthetic pathway in Corynebacterium glutamicum by targeted gene deletion and homologous complementation" JOURNAL OF BIOTECHNOLOGY, ELSEVIER SCIENCE PUBLISHERS B.V., AMSTERDAM, NL, Bd. 104, Nr. 1-3, 4. September 2003 (2003-09-04), Seiten 213-228, XP002329882 ISSN: 0168-1656
- LEE H -S ET AL: "Methionine biosynthesis and its regulation in Corynebacterium glutamicum: Parallel pathways of transsulfuration and direct sulfhydrylation." APPLIED MICROBIOLOGY AND BIOTECHNOLOGY, Bd. 62, Nr. 5-6, Oktober 2003 (2003-10), Seiten 459-467, XP002355864 ISSN: 0175-7598
- MAMPEL JOERG ET AL: "Single-gene knockout of a novel regulatory element confers ethionine resistance and elevates methionine production in Corynebacterium glutamicum" APPLIED MICROBIOLOGY AND BIOTECHNOLOGY, Bd. 68, Nr. 2, August 2005 (2005-08), Seiten 228-236, XP002355862 ISSN: 0175-7598

## Beschreibung

Die vorliegende Erfindung betrifft Mikroorganismen der Gattung Corynebacterium und Verfahren zur Herstellung von mindestens einer schwefelhaltigen Verbindung. Insbesondere betrifft die vorliegende Erfindung Verfahren zur Herstellung von L-Methionin oder L-Cystein in *Corynebacterium glutamicum.*

Aminosäuren werden als Zusatzstoffe in der Ernährung von Tieren und Menschen zu unterschiedlichen Zwecken verwendet. Beispielsweise wird Glutamat typischerweise als Geschmacksverstärkungsmittel benützt. Methionin ist eine essentielle Aminosäure, die mit der Nahrung aufgenommen werden muss. Sowohl Methionin als auch andere schwefelhaltige Aminosäuren wie Cystein sind nicht nur für die Proteinbiosynthese essentiell, sondern dienen auch als Vorläufer für verschiedene Metabolite wie Glutathion, S-Adenosylmethionin und Biotin und darüber hinaus als Methylgruppen-Donoren in verschiedenen zellulären Prozessen.

Der weltweite Verbrauch von Aminosäuren wird gegenwärtig auf über zwei Millionen Tonnen geschätzt. Der jährliche Bedarf an Aminosäuren wie Glutamat oder Futterzusatzstoffen, die hauptsächlich aus L-Lysin, L-Methionin und L-Threonin bestehen, wird auf mehr als eine Million Tonnen pro Aminosäure geschätzt. Der jährliche Bedarf an Aminosäuren, die in pharmazeutischen Produkten verwendet werden, beträgt alleine 15.000 Tonnen (Kusumoto, I. (2001), J. Nutr., 131, 2552S-2555S).

Während zu Beginn des letzten Jahrhunderts Aminosäuren hauptsächlich chemisch hergestellt oder durch Extraktion gewonnen wurden, erfolgt die Produktion bestimmter Aminosäuren wie z.B. Glutamat mittlerweile hauptsächlich durch Fermentationsverfahren. Dabei haben sich bestimmte Mikroorganismen wie z.B. *Escherichia coli* und *Corynebacterium glutamicum* als besonders geeignet zur fermentativen Herstellung von Aminosäuren erwiesen. Die Herstellung von Aminosäuren durch Fermentation hat insbesondere den Vorteil, dass ausschließlich die natürlich verwendbaren L-Aminosäuren hergestellt werden, wohingegen bei chemischen Synthesen in der Regel ein racemisches Gemisch entsteht, wodurch häufig eine zeitintensiven und kostenträchtigen Aufarbeitung erforderlich wird.

Insbesondere besteht ein Bedarf an sog. schwefelhaltigen Verbindungen wie Methionin, Homocystein, S-Adenosylmethionin, Glutathion, Cystein, Biotin, Thiamin, Liponsäure etc., die in vielen Industriezweigen, einschließlich der Nahrungsmittel-, Futtermittel-, Kosmetik- und pharmazeutischen Industrie verwendet werden. Diese Substanzen, die auch als schwefelhaltige Feinchemikalien bezeichnet werden, umfassen organische Säuren, proteinogene und nichtproteinogene Aminosäuren, Vitamine und Co-Faktoren.

Es wurde versucht, Mikroorganismen wie *E. coli* und *C. glutamicum* zur Herstellung der vorstehend genannten schwefelhaltigen Verbindungen einzusetzen. Aufgrund der großen Bedeutung der schwefelhaltigen Verbindungen wurden insbesondere Versuche zur Verbesserung der Herstellungsverfahren dieser Mikroorganismen, z.B. durch fermentationstechnische Maßnahmen, wie Rühren und Versorgung mit Sauerstoff, Zusammensetzung der Nährmedien etc., unternommen.

Daneben wurde versucht, über klassische Stammselektion Mutantenstämme der genannten Mikroorganismen zu entwickeln, die die genannten schwefelhaltigen Verbindungen in wesentlich größerem Maßstab als der jeweilige nicht-mutierte Wildtyp produzieren.

Die Verwendung von Mikroorganismen wie *E. coli* oder C. *glutamicum* hat sich dabei insbesondere bei der fermentativen Herstellung im industriellen Großmaßstab von schwefelhaltigen Aminosäuren wie Methionin und Cystein als schwierig erwiesen. Es besteht jedoch ein großer Bedarf gerade an diesen Aminosäuren, da sie unter anderem Vorläuferverbindungen für einige weitere der vorstehend genannten schwefelhaltigen Verbindungen sind.

Der Grund dafür, dass sich die fermentative Herstellung in Mikroorganismen für Methionin und Cystein im industriellen Maßstab bislang nicht als wirtschaftlich interessant erwiesen hat, liegt höchstwahrscheinlich in der Biosynthese bzw. den Stoffwechselwegen, die zur Herstellung von Methionin und Cystein führen. Die Biosynthese von Cystein und Methionin in *E. coli* ist allgemein bekannt (siehe z.B. Voet und Voet (1995) Biochemistry, John Wiley and Sons, Inc. USA). Die Biosynthese von Methionin und Cystein in *Corynebacterium glutamicum* ist Gegenstand intensiver Forschung und zuletzt in Rückert et al. (Rückert et al. (2003), J. of Biotechnology, 104, 213-228) sowie Lee et al. (Lee et al. (2003), Appl. Microbiol. Biotechnol., 62, 459-467) beschrieben worden.

Der Schlüsselschritt in der Biosynthese von Methionin ist der Einbau von Schwefel in das Kohlenstoffgerüst. Bei der Schwefelquelle handelt es sich regelmäßig um Sulfat, das von den Mikroorganismen aufgenommen, aktiviert und reduziert werden muss. Diese Schritte führen zu einem Verbrauch von 7 Mol ATP und 8 Mol NADPH pro Mol Methionin (Neidhardt et al. (1990) Physiology of the bacterial cell: a molecular approach, Sunderland, Massachusetts, USA, Sinauer Associates, Inc.). Damit stellt Methionin die Aminosäure dar, deren Herstellung die Zelle am meisten Energie kostet.

Entsprechend sind von den Methionin-herstellenden Mikroorganismen Stoffwechselwege entwickelt worden, die unter einer strikten Regulationskontrolle bezüglich der Herstellung von Methionin, aber auch von Cystein stehen. Dies bedeutet, dass, z.B. durch Feedback-Regulationsmechanismen, die Methionin verwendenden Stoffwechselwege in ihrer Aktivität herunterreguliert werden, wenn die Zelle ausreichend Methionin hergestellt hat.

Dies hat dazu geführt, dass bislang Methionin als einzige Aminosäure im industriellen Maßstab ausschließlich chemisch hergestellt wird, obwohl dies die anschließende Enantiomerentrennung der L- und D-Formen von Methionin erfordert.

Über Methionin-produzierende Varianten von *Corynebacterium glutamicum* ist daher nur wenig bekannt. Derartige Mutanten wurden bereits 1975 identifiziert (Kase et al. (1975) Agric. Biol. Chem., 39, 153-160). Die dort beschriebenen Stämme sind jedoch für die industrielle Herstellung von Methionin in Mikroorganismen nicht interessant. Verbesserungen wurden seitdem nicht erzielt.

Die Ansätze im Stand der Technik zur Herstellung von Mikroorganismen, die eine erhöhte Produktion von Methionin erlauben, haben sich bislang darauf konzentriert, die Gene, die in den Biosyntheseweg von Methionin und anderen schwefelhaltigen Verbindungen involviert sind, zu identifizieren und dann durch Überexpression oder Repression, je nach der jeweiligen Funktion, der Gene die Produktion an Methionin oder der anderen Verbindungen zu erhöhen.

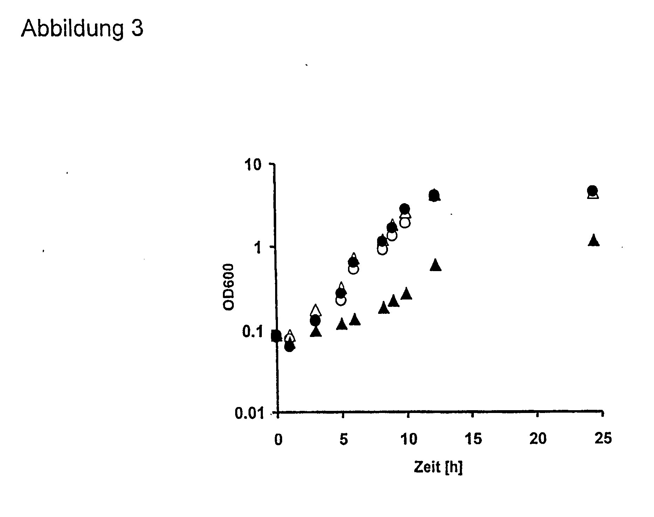

Solche Versuche sind z.B. in WO 02/10209 beschrieben. Als problematisch hat sich dabei herausgestellt, dass dort die Überexpression bzw. Repression nur einzelner Gene beschrieben wird, um die Herstellung z.B. von Methionin zu erhöhen. Da damit aber in der Regel nur der Kontrollmechanismus für einen Syntheseschritt der Biosynthese z.B. von Methionin ausgeschaltet wird und die anderen Regulationsmechanismen unberührt bleiben, kann auf diese Weise eine erhöhte Produktion von schwefelhaltigen Verbindungen nur in beschränktem Maß erreicht werden (siehe auch Abbildung 3 in Lee et al. *vide supra*). Durch die Überexpression eines einzelnen Gens, das in die Biosynthese von schwefelhaltigen Verbindungen involviert ist, werden daher in der Regel die oben erwähnten Regulationsmechanismen nicht ausreichend genug entkoppelt. Die zwar erzielten, aber nur geringen Steigerungen des Gehalts an Methionin oder anderen schwefelhaltigen Verbindungen reichen nicht aus, um das Verfahren für die fermentative Herstellung in Mikroorganismen im industriellen Maßstab interessant zu machen.

Dabei tritt bei der Herstellung von schwefelhaltigen Verbindungen wie z.B. Methionin in Mikroorganismen als besonderes Problem auf, dass die Gene des Biosynthesewegs von Methionin über das ringförmige Genom von Mikroorganismen verteilt sein können (siehe Rückert et al., *vide supra*). Beispiele für Mikroorganismen mit derart über das Genom verteilten Genen des Biosynthesewegs von Methionin sind u.a. *Corynebacterium glutamicum, Escherichia coli, Bacillus* wie *Bacillus subtilis, Serratia* wie *Serratia marcescens* und *Salmonella.* Die Verteilung der für die Methionin-Biosynthese verantwortlichen Gene kann als ein Hinweis darauf gesehen werden, dass diese Gene z.B. nicht in einem Operon, d.h. in einer gemeinsamen Regulationseinheit organisiert sind. Wäre dies der Fall, könnten z.B. durch Beeinflussung, d.h. Überexpression oder Repression des molekularen Schalters, der dieses Operon reguliert, mehrere Regulationsmechanismen auf einmal entkoppelt werden und so eine effizientere Steigerung der von Mikroorganismen produzierten Methioninmenge erreicht werden. Dass solche Operons grundsätzlich trotz der Verteilung der in der Biosynthese von Methionin involvierten Gene im Genom von *Corynebacterium glutamicum* existieren können, wurde vor kurzem gezeigt (Rey et al. (2003), J. Biotechnol., 103, 51-65).

Rey et al. konnten zeigen, dass es sich bei dem Protein McbR um einen Transkriptionsrepressor handelt, der die Expression der in die Biosynthese von Methionin involvierten Gene *metY* (kodierend für O-Acetyl-L-Homoserinsulfhydrylase), *metK* (kodierend für S-Adenosyl-Methioninsynthetase), *hom* (kodierend für Homoserindehydrogenase, *cysK* (kodierend für L-Cysteinsynthase), *cysI* (kodierend für NADPH-abhängige Sulfidreduktase) sowie *ssuD* (kodierend für Alkansulfonatmonooxygenase) reguliert. Die vorgenannten Enzyme sind alle in die Biosynthese von Methionin und/oder Cystein involviert und die Autoren konnten entsprechend zeigen, dass in einem Knockout-Stamm von *C. glutamicum* für McbR erhöhte Mengen an Methionin produziert werden.

Die für den Transkriptionsregulator McbR kodierende DNA Sequenz besitzt eine Identität von 46% zu der Sequenz SEQ ID NO: 1, die weiter beschrieben wird.

Der in der US 2003/16227 A und WO 2004/050694 A beschriebene negative Regulator RXA00655 entspricht einem funktionell homologen Molekül, da er die Produktion von Methionin in Corynebacterium glutamicum negativ beeinflusst. Seine kodierende DNA Sequenz ist zu 42,5 % identisch mit der Sequenz SEQ ID NO:1.

Angesichts dieser Ergebnisse besteht ein starkes Interesse daran, weitere Faktoren zu identifizieren, die in die zentrale Regulation der Biosynthesewege für schwefelhaltige Verbindungen in Mikroorganismen involviert sind, da es durch die Überexpression bzw. Repression (je nach Funktion) solcher zentralen Regulationsschalter möglich sein sollte, Mikroorganismen herzustellen, die signifikant erhöhte Mengen an schwefelhaltigen Verbindungen produzieren.

Es ist daher eine Aufgabe der vorliegenden Erfindung, Mikroorganismen zur Verfügung zu stellen, in denen schwefelhaltige Verbindungen hergestellt werden können.

Insbesondere ist es auch eine Aufgabe der vorliegenden Erfindung, Mikroorganismen und Verfahren zur Herstellung von schwefelhaltigen Verbindungen wie Methionin oder Cystein in Mikroorganismen der Gattung *Corynebacterium* zur Verfügung zu stellen, bei denen es durch Ausschalten eines zentralen Regulationselements der Biosynthesewege der schwefelhaltigen Verbindungen ermöglicht wird, signifikant erhöhte Mengen der vorstehend genannten schwefelhaltigen Verbindungen zu erhalten.

Darüber hinaus werden Nukleinsäuresequenzen beschrieben, die verwendet werden können, um mutierte Organismen herzustellen, die im Vergleich zu ihrem jeweiligen Wildtyp erhöhte Mengen an schwefelhaltigen Verbindungen wie z.B. Methionin und/oder Cystein produzieren.

Bevorzugte Ausführungsformen der Erfindung sind in den Unteransprüchen beschrieben.

Im Rahmen der vorliegenden Erfindung konnte durch einen neuen Selektions- und Mutationsmechanismus in C. *glutamicum* ein Faktor, nämlich NCg12640 (Genbank accession number der Entrez-Datenbank, http://www.ncbi.nlm.nih.gov, SEQ ID No. 1) identifiziert werden, der einen zentralen Regulationsschalter in der Regulation von schwefelhaltigen Verbindungen in C. *glutamicum* und insbesondere von Methionin darstellt. Da eine Homologie-Suche mit der durch NCg12640 kodierten Aminosäuresequenz (Genbank accession number: NP_601931, SEQ ID No. 2) mit dem BLAST-Programm am NCBI (http:/www.ncbi.nlm.nih.gov) ergeben hat, dass mehrere Homologe in verschiedenen anderen Mikroorganismen wie z.B. E. *coli* bestehen, kann davon ausgegangen werden, dass angesichts der Konservierung von Biosynthesewegen für Aminosäuren und schwefelhaltige Verbindungen insbesondere zwischen den verschiedenen Mikroorganismen dieser Faktor auch in den anderen Mikroorganismen in die zentrale Regulation der Biosynthesewege von schwefelhaltigen Verbindungen involviert ist.

Wie im Rahmen der vorliegenden Erfindung gezeigt wird, können Corynebacterien bei denen der Gehalt von Nukleinsäuren, die identisch mit Nukleinsäuren mit der Sequenz SEQ ID No. 1 sind, im Vergleich zum Wildtyp erniedrigt ist, zur Herstellung von schwefelhaltigen Verbindungen verwendet werden.

Daher betrifft ein Gegenstand der vorliegenden Erfindung ein Corynebacterium bei dem im Vergleich zum Wildtyp des Mikroorganismus der Gehalt von Proteinen erniedrigt ist, die durch Nukleinsäuren kodiert sind, die identisch mit Nukleinsäuren mit der Sequenz SEQ ID No. 1 sind. Im folgenden wird dieser Gegenstand der vorliegenden Erfindung auch dadurch beschrieben, dass im Vergleich zum Wildtyp des Mikroorganismus der Gehalt von Nukleinsäuren, die identisch mit Nukleinsäuren mit der Sequenz SEQ ID No. 1 sind, erniedrigt ist.

Ein weiterer Gegenstand der vorliegenden Erfindung betrifft die oben genannten Corynebacterien, die aufgrund des/der im Vergleich zum Wildtyp erniedrigten Gehalts von Proteinen, die durch Nukleinsäuren kodiert sind, die identisch mit Nukleinsäuren mit der Sequenz SEQ ID No. 1 sind, bzw. aufgrund des/der im Vergleich zum Wildtyp erniedrigten Gehalts/Aktivität von Nukleinsäuren, die identisch mit Nukleinsäuren mit der Sequenz SEQ ID No. 1 sind, in der Lage sind, schwefelhaltige Verbindungen wie L-Methionin, L-Cystein, L-Homocystein, L-Cystathionin, S-Adenosyl-L-Methionin, Glutathion, Biotin, Thiamin und/oder Liponsäure, bevorzugt L-Methionin und/oder L-Cystein herzustellen.

Ein weiterer Gegenstand der vorliegenden Erfindung betrifft Corynebacterien, bei denen im Vergleich zum Wildtyp der Gehalt und/oder die Aktivität von Proteinen, die durch Nukleinsäuren kodiert sind, die mindestens zu 95% identisch mit Nukleinsäuren mit der Sequenz SEQ ID No 1 sind, durch Disruption und/oder Deletion der entsprechenden genomischen Nukleinsequenz(en) erniedrigt worden ist.

Ein weiterer Gegenstand der vorliegenden Erfindung betrifft Mikroorganismen, bei denen im Vergleich zum Wildtyp der Gehalt der Nukleinsäuren, die identisch mit Nukleinsäuren mit der Sequenz SEQ ID No. 1 sind, bzw. der Gehalt der durch diese Nukleinsäuren kodierten Proteine dadurch erniedrigt worden ist, dass in die genomischen Nukleinsäuresequenzen Mutationen eingeführt sind, die zur Expression von nicht-funktionellen Formen der durch die vorstehend genannten Nukleinsäuren kodierten Proteine führen.

Ein weiterer Gegenstand der vorliegenden Beschreibung betrifft Mikroorganismen, bei denen im Vergleich zum Wildtyp der Gehalt der Nukleinsäuren, die identisch mit Nukleinsäuren mit der Sequenz SEQ ID No. 1 sind, durch Antisense-Verfahren, durch Expression eines Ribozyms, das für die mRNA dieser Nukleinsäuren spezifisch ist, oder durch Expression von Ribonuklease-P-Konstrukten erniedrigt worden ist.

Ein weiterer Gegenstand der vorliegenden Beschreibung betrifft Mikroorganismen, bei denen im Vergleich zum Wildtyp der Gehalt der Nukleinsäuren, die identisch mit Nukleinsäuren mit der Sequenz SEQ ID No. 1 sind, bzw. der Gehalt der durch diese Nukleinsäuren kodierten Proteine dadurch erniedrigt worden ist, dass rekombinante Antikörper in den Mikroorganismen exprimiert werden, die für die durch die Nukleinsäuren kodierten Proteine spezifisch sind und deren Aktivität blockieren bzw. inhibieren.

Ein weiterer Gegenstand der vorliegenden Beschreibung betrifft Mikroorganismen, bei denen im Vergleich zum Wildtyp der Gehalt und/oder die Aktivität der Nukleinsäuren, die identisch mit Nukleinsäuren mit der Sequenz SEQ ID No. 1 sind, bzw. der Gehalt und/oder die Aktivität der durch diese Nukleinsäuren kodierten Proteine dadurch erniedrigt worden ist, dass eine nicht-funktionelle Form der durch die Nukleinsäuren kodierten Proteine in den Zellen überexprimiert wird.

Ein weiterer Gegenstand der vorliegenden Beschreibung betrifft Mikroorganismen, bei denen im Vergleich zum Wildtyp zusätzlich zu der Erniedrigung des Gehalts der Nukleinsäuren, die identisch mit Nukleinsäuren mit der Sequenz SEQ ID No. 1 sind, bzw. der Erniedrigung des Gehalts der durch diese Nukleinsäuren kodierten Proteine der Gehalt von Nukleinsäuren, die für McbR aus *C. glutamicum* kodieren, erniedrigt worden ist.

Ein Gegenstand der Erfindung betrifft somit Mikroorganismen, bei denen im Vergleich zum Wildtyp zusätzlich der Gehalt von Nukleinsäuren, die identisch mit Nukleinsäuren mit der Sequenz SEQ ID No. 3 sind, bzw. der Gehalt und/oder die Aktivität von Proteinen, die zu Proteinen mit der Sequenz SEQ ID No. 4 identisch sind, erniedrigt worden ist. Die Erniedrigung des Gehalts oder der Aktivität kann dabei in gleicher Weise wie vorstehend beschrieben erfolgen.

Eine besonders bevorzugte Ausführungsform der erfindungsgemäßen Mikroorganismen betrifft Mikroorganismen, bei denen die Expression der vorgenannten Nukleinsäuresequenzen bzw. dadurch kodierten Proteine im Wesentlichen vollständig unterdrückt ist.

Ein weiterer Gegenstand der vorliegenden Beschreibung betrifft Mikroorganismen, bei denen im Vergleich zum Wildtyp neben der Erniedrigung des Gehalts der Nukleinsäuren, die identisch mit Nukleinsäuren mit der Sequenz SEQ ID No. 1 sind, bzw. der dadurch kodierten Proteine, der Gehalt mindestens einer weiteren Nukleinsäure, die für ein Genprodukt des Biosynthesewegs der gewünschten schwefelhaltigen Verbindung kodiert, erhöht ist. In solchen Mikroorganismen können die letztgenannten Nukleinsäuren auch derart mutiert sein, dass das durch die Nukleinsäure kodierte Protein durch Stoffwechselmetabolite nicht in seiner Aktivität beeinflusst wird. In diesen Mikroorganismen können der Gehalt an McbR aus C. *glutamicum* bzw. an Homologen wie oben erwähnt dazu erniedrigt sein.

In gleicher Weise betrifft ein weiterer Gegenstand der vorliegenden Beschreibung Mikroorganismen, in denen neben der Erniedrigung des Gehalts von Nukleinsäuren, die identisch mit Nukleinsäuren mit der Sequenz SEQ ID No. 1 sind, bzw. von dadurch kodierten Proteinen zusätzlich der Gehalt mindestens einer weiteren Nukleinsäure, die für ein Genprodukt des Biosynthesewegs der gewünschten schwefelhaltigen Verbindung kodiert, im Vergleich zum Wildtyp erniedrigt ist. In diesen Mikroorganismen können der Gehalt an McbR aus *C*. *glutamicum* bzw. an Homologen wie oben erwähnt dazu erniedrigt sein.

Ein weiterer Gegenstand der vorliegenden Erfindung betrifft Verfahren zur Herstellung von schwefelhaltigen Verbindungen in Mikroorganismen, die das Kultivieren eines erfindungsgemäßen Corynebacteriums umfassen. Ein besonders bevorzugtes Verfahren zur Herstellung von L-Methionin und/oder L-Cystein benutzt dabei den Mikroorganismus *Corynebacterium glutamicum,* bei dem der Gehalt von Nukleinsäuren, die identisch mit Nukleinsäuren mit der Sequenz SEQ ID No. 1 sind, bzw. der durch diese Nukleinsäuren kodierten Proteine im Vergleich zum Wildtyp erniedrigt wird.

Ein weiterer Gegenstand der vorliegenden Erfindung betrifft ein Verfahren zur Herstellung von L-Methionin und/oder L-Cystein unter Verwendung von Corynebacterien, in dem zusätzlich zu der Erniedrigung des Gehalts und/oder der Aktivität der Nukleinsäuren, die identisch mit Nukleinsäuren mit der Sequenz SEQ ID No. 1 sind, bzw. der durch diese Nukleinsäuren kodierten Proteine der Gehalt von weiteren oben genannten Nukleinsäuren in der beschriebenen Weise erhöht oder erniedrigt ist.

Ein weiterer Gegenstand der vorliegenden Beschreibung betrifft die Verwendung der oben genannten Nukleinsäuren zur Herstellung von Mikroorganismen, die zur Herstellung von schwefelhaltigen Verbindungen verwendet werden können.

Im Rahmen der vorliegenden Erfindung konnte ein Regulator der Methionin-Biosynthese in C. *glutamicum* identifiziert werden. Dies wurde durch Anwendung einer neuartigen Selektions- und Mutationsstrategie möglich, die sich signifikant von den früher im Stand der Technik unternommenen Versuchen zur Identifizierung von Methionin-überproduzierenden *C. glutamicum*-Stämmen unterscheidet (siehe Beispiele).

Dabei konnte im Rahmen der vorliegenden Erfindung erstmals gezeigt werden, dass die Nukleinsäuresequenz mit der SEQ ID No. 1 (Genbank accession number: NCg12640) für ein Protein mit der Aminosäuresequenz SEQ ID No. 2 (Genbank accession number: NP_601931) kodiert, das aktiv in die Regulation des Biosynthesewegs von Methionin in *C. glutamicum* involviert ist. Weiterhin konnte im Rahmen der vorliegenden Erfindung erstmals gezeigt werden, dass bei Deletion bzw. funktioneller Disruption der Nukleinsäuresequenz mit der SEQ ID No. 1 in *C. glutamicum* Stämme erhalten werden, die aufgrund der ausgeschalteten Regulation des durch die SEQ ID No. 1 kodierten Proteins Methionin in verstärktem Maße produzieren.

Eine BLAST-Recherche am NCBI zeigte, dass Homologe zu NCg12640 aus *C. glutamicum* in verschiedensten Mikroorganismen existieren. Da die Biosynthesewege für schwefelhaltige Verbindungen in verschiedensten Organismen konserviert sind, wird davon ausgegangen, dass Mikroorganismen, in denen im Vergleich zum Wildtyp der Gehalt und/oder die Aktivität von Nukleinsäuren, die identisch mit oder funktionell homolog zu einer Nukleinsäure mit der Sequenz SEQ ID No. 1 sind, erniedrigt ist, ebenfalls in der Lage sind, Methionin und/oder andere schwefelhaltige Verbindungen wie Cystein in verstärktem Maße zu produzieren.

Unter NCg12640 wird erfindungsgemäß ein Nukleinsäuremolekül mit der SEQ ID No. 1 verstanden. Das durch ein solches Nukleinsäuremolekül kodierte Protein hat die Sequenz SEQ ID No. 2.

Unter funktionellen Homologen zu der Nukleinsäure mit der Sequenz SEQ ID No. 1 bzw. dem Protein mit der Aminosäuresequenz No. 2 werden solche Nukleinsäuremoleküle bzw. Proteine verstanden, deren Sequenz eine signifikante Homologie zu der Nukleinsäure mit der Sequenz SEQ ID No. 1 bzw. dem Protein mit der Aminosäuresequenz SEQ ID No. 2 aufweisen.

Unter dem Gehalt an NCg12640 bzw. dem Gehalt an Nukleinsäuren, die identisch mit oder funktionell homolog zu einer Nukleinsäure mit der Sequenz SEQ ID No. 1 sind, wird die Menge an diesen Nukleinsäuren bzw. an den durch diese Nukleinsäuren kodierten Proteinen verstanden, wie sie für den entsprechenden Mikroorganismus bestimmt werden kann.

Unter der Aktivität von NCg12640 bzw. von Nukleinsäuren, die identisch mit einer Nukleinsäure mit der Sequenz SEQ ID No. 1 sind, wird erfindungsgemäß die zelluläre Aktivität der durch diese Nukleinsäuresequenzen kodierten Proteine verstanden. Die zelluläre Funktion von Proteinen, die durch Nukleinsäuren kodiert sind, die identisch mit einer Nukleinsäure mit der Sequenz SEQ ID No. 1 sind, ist in der Regulation der Biosynthese von schwefelhaltigen Verbindungen und insbesondere Methionin zu sehen. Ob eine zu den Nukleinsäuren mit den Sequenzen SEQ ID No. 1 und 2 homologe Nukleinsäure über die gleiche Aktivität wie die Nukleinsäure mit den Sequenzen SEQ ID No. 1 bzw. 2 in *C. glutamicum* verfügen, kann in einfacher Weise bestimmt werden. Dazu wird in den entsprechenden Mikroorganismen die entsprechende genomische Nukleinsäuresequenz deletiert und ermittelt, ob der Mikroorganismus trotz Zugabe des Strukturanalogons von Methionin wie z.B. D,L-Ethionin noch in der Lage ist, Methionin oder andere schwefelhaltige Verbindungen zu produzieren.

Unter einem gegenüber dem Wildtyp veränderten Gehalt an Nukleinsäuren, die identisch mit einer Nukleinsäure mit der Sequenz SEQ ID No. 1 sind, wird erfindungsgemäß eine im Vergleich zum Wildtyp erniedrigte Menge an diesen Nukleinsäuren bzw. an den durch diese Nukleinsäure kodierten Proteinen verstanden. Die Erniedrigung der Menge an diesen Nukleinsäuren bzw. an den durch diese Nukleinsäure kodierten Proteinen wird im Allgemeinen durch eine Erniedrigung des Gehalts an den endogenen Nukleinsäuren, bei denen es sich entweder um Nukleinsäuren mit der Sequenz SEQ ID No. 1 Nukleinsäuren handelt, erzielt.

Unter einem Wildtyp wird erfindungsgemäß der entsprechende, nicht genetisch veränderte Ausgangsorganismus verstanden.

Die Erniedrigung der Aktivität der Nukleinsäuren, die identisch mit zu einer Nukleinsäure mit der Sequenz SEQ ID No. 1 sind, bzw. der durch diese Nukleinsäuren kodierten Proteine kann durch eine Erniedrigung der Menge bzw. des Gehalts der endogenen Nukleinsäuren bzw. der endogenen durch diese Nukleinsäuren kodierten Proteine erzielt werden. Daneben wird erfindungsgemäß unter der Erniedrigung der Aktivität an den genannten Nukleinsäuren bzw. den durch diese Nukleinsäuren kodierten Proteine verstanden, dass die Aktivität an endogenen Nukleinsäuren bzw. an durch diese endogenen Nukleinsäuren kodierten Proteinen unverändert bleibt, aber die Interaktion der durch die Nukleinsäuren kodierten Proteine mit ihren zellulären Bindungspartnern z.B. durch die Expression nicht funktioneller Formen von NP_601931 bzw. Homologen davon oder dafür spezifischen Antikörpern signifikant inhibiert wird.

Vorzugsweise beträgt die in den erfindungsgemäßen Corynebacterien auftretende Erniedrigung des Gehalts und/oder der Aktivität an Nukleinsäuren, die identisch mit einer Nukleinsäure mit der Sequenz SEQ ID No. 1 sind, bzw. an den durch diese Nukleinsäuren kodierten Proteine mindestens 95% bevorzugt mindestens 98% und am meisten bevorzugt 100%.

Der Begriff Repression der Expression von Nukleinsäuren, die identisch mit einer Nukleinsäure mit der Sequenz SEQ ID No. 1 sind, bzw. von durch diese Nukleinsäuren kodierten Proteinen ist erfindungsgemäß synonym mit dem Begriff der Erniedrigung des Gehalts und/oder der Aktivität an diesen Nukleinsäuren bzw. an durch diese Nukleinsäuren kodierten Proteinen.

Wie bereits vorstehend angesprochen wurde, konnten durch eine BLAST-Analyse am NCBI Homologe von NCg12640 in verschiedensten Organismen identifiziert werden. Da in Zukunft zunehmend weitere Sequenzdaten für unterschiedlichste Mikroorganismen zur Verfügung stehen werden, schließt die vorliegende Erfindung auch solche Mikroorganismen ein, in denen Nukleinsäuren bzw. dadurch kodierte Proteine bezüglich ihres Gehalts im Vergleich zum Wildtyp erniedrigt sind, sofern diese Nukleinsäuren bzw. die durch diese Nukleinsäuresequenzen kodierten Aminosäuresequenzen eine signifikante oder wesentliche Sequenzhomologie zu den Nukleinsäuren mit der Sequenz SEQ ID No. 1 bzw. Proteinen mit der Sequenz SEQ ID No. 2 zeigen.

Unter Identität zwischen zwei Nukleinsäuren bzw. Proteinen wird die Identität der Nukleinsäuresequenzen bzw. Aminosäursequenzen über einen bestimmten Sequenzbereich verstanden, vorzugsweise die gesamte Sequenzlänge, insbesondere die Identität, die durch Vergleich mit Hilfe der Lasergene-Software der Firma DNA Star Inc., Madison, Wisconsin (USA) unter Anwendung der CLUSTAL-Methode (Higgins et al., (1989), Comput. Appl. Biosci., 5 (2), 151) berechnet wird. Homologien können ebenfalls mit Hilfe der Lasergene-Software der Firma DNA Star Inc., Madison, Wisconsin (USA) unter Anwendung der CLUSTAL-Methode (Higgins et al., 1989), Comput. Appl. Biosci., 5 (2), 151) berechnet werden.

Nukleinsäuremoleküle sind identisch, wenn sie gleiche Nukleotide in gleicher 5'-3'-Reihenfolge aufweisen.

Aminosäuremoleküle sind identisch, wenn sie von N- zum C-Terminus gleiche Aminosäuren in gleicher Reihenfolge aufweisen.

Unter signifikanter bzw. wesentlicher Sequenzhomologie wird erfindungsgemäß allgemein verstanden, dass die Nukleinsäuresequenz eines DNA-Moleküls bzw. die Aminosäuresequenz eines Proteins zu mindestens 95% und am meisten bevorzugt zu mindestens 98% zu den Nukleinsäure- bzw. Aminosäuresequenzen SEQ ID No. 1 bzw. SEQ ID No. 2 oder deren funktionell äquivalenten Teilen identisch ist. Vorzugsweise wird die Homologie über die gesamte Sequenzlänge von SEQ ID No.1 bzw. SEQ ID No. 2 bestimmt.

Vorzugsweise wird die Homologie somit über den gesamten Aminosäure- bzw. Nukleinsäuresequenzbereich berechnet. Neben den oben genannten Programmen stehen dem Fachmann für den Vergleich verschiedener Sequenzen noch weitere Programme zur Verfügung, die auf verschiedenen Algorithmen beruhen. Dabei liefern die Algorithmen von Needleman und Wunsch oder Smith und Waterman besonders zuverlässige Ergebnisse. Für die Sequenzvergleiche kann z.B. auch das Programm Pile Aupa verwendet werden (J. Mol. Evolution. (1987), 25, 351-360; Higgins et al., (1989) Cabgos, 5, 151-153) oder die Programme Gap und Best Fit (Needleman und Wunsch, (1970), J. Mol. Biol., 48, 443-453 sowie Smith und Waterman (1981), Adv., Appl. Math., 2, 482-489), die im GCG-Software-Paket der Genetics Computer Group (575 Science Drive, Madison, Wisconsin, USA 53711) enthalten sind.

Eine Ausführungsform der vorliegenden Erfindung betrifft die Verwendung der vorstehend genannten Nukleinsäuren zur Herstellung von Mikroorganismen, die in der Lage sind, im Vergleich zum Wildtyp erhöhte Mengen an schwefelhaltigen Verbindungen und insbesondere an Methionin und/oder Cystein zu produzieren. Dabei ist dem Fachmann klar, dass er zur Herstellung eines Mikroorganismus, der in der Lage ist, erhöhte Mengen an schwefelhaltigen Verbindungen zu produzieren, jeweils den Gehalt bzw. die Aktivität der Nukleinsäure im Vergleich zum Wildtyp reduzieren wird, die in dem jeweiligen Organismus dem funktionellen Homolog zu NCg12640 aus *C. glutamicum* entspricht.

DNA-Sequenzen mit einer hohen Homologie, d.h. einer hohen Ähnlichkeit oder Identität sind *bona fide* Kandidaten für DNA-Sequenzen, die den Sequenzen SEQ ID No. 1 der erfindungsgemäßen Nukleinsäuren entsprechen. Diese Gensequenzen können durch Standardmethoden, wie z.B. PCR und Hybridisierung isoliert werden, und ihre Funktion kann durch entsprechende Enzymaktivitätstests und andere Experimente durch den Fachmann bestimmt werden. Homologievergleiche mit DNA-Sequenzen können erfindungsgemäß auch verwendet werden, um PCR-Primer zu entwickeln, indem zunächst die Regionen identifiziert werden, die in den DNA-Sequenzen von verschiedenen Organismen am meisten konserviert sind. Solche PCR-Primer können dann benutzt werden, um in einem ersten Schritt DNA-Fragmente zu isolieren, die Bestandteile von Nukleinsäuren sind, die zu den Nukleinsäuren der Erfindung homolog sind.

Es gibt eine Reihe von Suchmaschinen, die für solche Homologievergleiche bzw. -suchen verwendet werden können. Diese Suchmaschinen umfassen z.B. die CLUSTAL-Programmgruppe des BLAST-Programms, das durch das NCBI zur Verfügung gestellt wird.

Darüber hinaus ist dem Fachmann eine Reihe von experimentellen Methoden bekannt, mit denen DNA-Sequenzen aus verschiedensten Organismen isoliert werden können, die zu den erfindungsgemäßen Sequenzen homolog sind. Dazu gehört z.B. das Anfertigen und Screenen von cDNA-Bibliotheken mit entsprechend degenerierten Sonden.

Bei zur SEQ ID No. 2 homologen Sequenzen kann es sich z.B. um Sequenzen handeln, die für die folgenden Proteine kodieren: NP_601931.1; NP_739184.1; NP_940366.1; NP_962856.1; ZP_00226250.1; NP_214947.1; NP_334857.1; ZP_00058595.1; NP_925240.1; NP_969100.1; ZP_00019148.1; ZP_00199594.1; ZP_00015952.1; ZP_00160492.1; NP_770404.1; NP_866130.1; ZP_00026378.1; NP_280238.1; ZP_00029745.1; ZP_00226340.1; NP_882643.1; NP_459575.1; NP_879442.1; ZP_00213193.1; ZP 00221250.1; NP_806026.1; NP_521417.1; NP_455160.1; ZP_00185946.2; ZP_00203540.1; NP_415113.1; NP_752598.1; NP_286306.1; NP_902574.1; ZP_00052011.1; NP_706431.1; NP_822174.1; NP_745396.1; NP_739496.1 (bei den Nummern handelt es sich jeweils um *Genbank accession numbers*).

Wie bereits erwähnt, kann die Änderung des Gehalts und/oder der Aktivität von an Nukleinsäuren, die identisch mit einer Nukleinsäure mit der Sequenz SEQ ID No. 1 sind, bzw. von Proteinen, die durch diese Nukleinsäuren kodiert werden, auf verschiedene Weise erfolgen. Die Erniedrigung des Gehalts kann z.B. durch Ausschalten von stimulierenden Regulationsmechanismen auf Transkriptions-, Translations- und/oder Proteinebene oder durch Erniedrigung der Genexpression der jeweiligen Nukleinsäuren erfolgen.

Bei einer bevorzugten Ausführungsform wird die Expression der jeweiligen genomischen Sequenzen reduziert sein. Bevorzugt wird es sich um Mikroorganismen handeln, in denen die genomischen Sequenzen für die Nukleinsäuren, die identisch mit einer Nukleinsäure mit der Sequenz SEQ ID No. 1 sind, inaktiviert sind. Eine Inaktivierung dieser genomischen Sequenzen kann auf verschiedene Weisen erfolgen. Eine Möglichkeit ist es, die genomischen Sequenzen durch homologe Rekombination zu deletieren. Eine andere Möglichkeit ist es, in die genomischen Sequenzen Insertionen einzuführen, die wiederum die Expression der Nukleinsäuren verhindern bzw. zu nicht funktionellen Proteinen führen.

Unter nicht funktionellen Proteinen werden im Rahmen der vorliegenden Erfindung solche Proteine bzw. Proteinfragmente verstanden, bei denen durch Punktmutationen, Insertionen oder Deletionen die Struktur und damit die Funktion des Proteins mit der Aminosäuresequenz SEQ ID No. 2 derart verändert wird, dass diese Proteine nicht mehr als zentrale Regulationsschalter in den Biosynthesewegen von schwefelhaltigen Verbindungen und insbesondere Methionin fungieren.

Ob durch die Einführung von Insertion, Deletion oder Mutation in die genomischen Sequenzen von Nukleinsäuren, die identisch mit einer Nukleinsäure mit der Sequenz SEQ ID No. 1 sind, nicht-funktionelle Proteine bzw. Proteinfragmente erhalten werden, kann in einfacher Weise ermittelt werden. Dazu werden die Mikroorganismen, die die mutmaßlichen nicht-funktionellen Proteine bzw. Proteinfragmente exprimieren, daraufhin getestet, ob sie in Anwesenheit von Methionin-Strukturanaloga wie z.B. D, L-Ethionin in der Lage sind zu wachsen. Ist dies der Fall, handelt es sich um nicht-funktionelle Proteine bzw. Proteinfragmente.

Die Reduktion der Expression der genomischen Sequenzen, die zur SEQ ID No. 1 identisch bzw. funktionell homolog sind, durch Deletion der genomischen Sequenzen bzw. durch Einführen von Mutationen, Insertionen oder Deletionen, die zur nicht-funktionellen Proteinen bzw. Proteinfragmenten führen, wird im Allgemeinen als funktionelle Disruption der genomischen Sequenzen bezeichnet.

Die Deletion bzw. funktionelle Disruption von genomischen Segmenten durch homologe Rekombination kann dabei durch ein Verfahren umfassend die folgenden Schritte erfolgen:
a) Herstellen eines Vektors umfassend folgende Nukleinsäuresequenzen in 5'-3'-Orientierung:
   - eine in Mikroorganismen funktionelle Promotorsequenz,
   - operativ daran gebunden eine DNA-Sequenz, die identisch oder homolog zu dem 5'-Ende der Nukleinsäuresequenz ist, die zu der Sequenz mit der SEQ ID No. 1 identisch oder funktionelle homolog ist,
   - operativ daran gebunden eine DNA-Sequenz, die für ein Resistenzgen kodiert,
   - operativ daran gebunden eine DNA-Sequenz, die identisch oder homolog zu dem 3'-Ende der Nukleinsäuresequenz ist, die zu der Sequenz mit der SEQ ID No. 1 identisch ist,
   - operativ daran gebunden eine in Mikroorganismen funkionelle Terminationssequenz; und
b) Übertragen des Vektors aus a) in den Mikroorganismus und gegebenenfalls Intergrieren des Vektors in dessen Genom.

Eine Möglichkeit zur homologen Rekombination kann den nachstehend dargestellten Beispielen entnommen werden. Dem Fachmann ist bekannt, dass für eine solche homologe Rekombination als Vektoren übliche Plasmide verwendet werden können, wie sie z.B. zur Überexpression von Nukleinsäuresequenzen in Mikroorganismen bekannt sind. Als Resistenzgene werden typischerweise Resistenzen für Antibiotika verwendet werden (siehe unten).

Unter einer operativen Verknüpfung versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Aktivierungssequenzen, Enhancer und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Sollen in die genomischen Sequenzen Punktmutationen, Insertionen oder Deletionen eingeführt werden, die zu nicht-funktionellen Proteinen bzw. Proteinfragmenten führen, geschieht dies üblicherweise anhand von Techniken, wie sie aus dem Stand der Technik bekannt sind (siehe u.a. Sambrook et al., Molecular Cloning: A Laboratory Manual, (2001), 3. Auflage, Cold Spring Harbor Laboratory Press, Cold Spring Harbor, USA sowie die sog. Quikchange Mutagenese-Methode von Stratagene, La Jolla, USA). Weitere Verfahren zur Einführung von Punktmutationen, Insertionen oder Deletionen in genomische Sequenzen von Mikroorganismen sind u.a. in Jäger et al., (1992) J. Bacteriol. 174, 5462-5465 und WO 02/070685 beschrieben.

Weitere Möglichkeiten zur Reduktion des Gehalts und/oder der Aktivität der vorstehend genannten Nukleinsäuren kann z.B. durch eine Antisense-Strategie erfolgen. Dazu kann z.B. ein Verfahren verwendet werden, das die folgenden Schritte umfasst:
a) Herstellen eines Vektors, umfassend folgende Nukleinsäuresequenzen in 5' -3' -Orientierung:
   - eine in dem jeweiligen Mikroorganismus funktionelle Promotersequenz,
   - operativ daran gebunden eine antisense-Sequenz zu SEQ ID No.1,
   - operativ daran gebunden eine in dem jeweiligen Mirkoorganismus funktionelle Terminationssequenz; und
b) Übertragen des Vektors aus a) auf den Mikrooganismus und gegebenenfalls Integrieren des Vektors in dessen Genom.

Bei einer weiteren Ausführungsform werden zur Herstellung der erfindungsgemäßen Mikroorganismen Vektoren verwendet, die eine DNA-Sequenz enthalten, die für ein Ribozym kodiert, das spezifisch die mRNA der Nukleinsäuren, die identisch mit einer Nukleinsäure mit der Sequenz SEQ ID No. 1 sind, erkennt. Dem Fachmann ist bekannt, wie Ribozyme, die gegen eine bestimmte mRNA gerichtete Endonukleaseaktivität besitzen, hergestellt werden können. Im Detail ist dies z.B. in Steineke et al., (Steineke et al. (1992) EMBO J., 11, 1525) beschrieben. Im Rahmen dieser Erfindung sind mit dem Begriff Ribozym auch solche RNA-Sequenzen gemeint, die neben dem eigentlichen Ribozym noch Führungssequenzen umfassen, die zu der mRNA der Nukleinsäuren, die identisch mit oder funktionell homolog zu einer Nukleinsäure mit der Sequenz SEQ ID No. 1 sind, komplementär sind und so das mRNA-spezifische Ribozym noch gezielter zum mRNA-Substrat des Ribozyms geführt wird.

Ein solches Verfahren umfasst z.B. die folgenden Schritte:
a) Herstellen eines Vektors, umfassend folgende Nukleinsäuresequenzen in 5'-3'-Orientierung:
   - eine in dem jeweiligen Mikroorganismus funktionelle Promotersequenz,
   - operativ daran gebunden eine DNA-Sequenz, die für ein Ribozym kodiert, das spezifisch die mRNA einer Nukleinsäure der SEQ ID No. 1,
   - operativ daran gebunden eine in dem jeweiligen Mirkoorganismus funktionelle Terminationssequenz; und
b) Übertragen des Vektors aus a) auf den Mikrooganismus und gegebenenfalls Integrieren des Vektors in dessen Genom.

Eine weitere Alternative zur Herstellung von erfindungsgemäßen Mikroorganismen bietet die Übertragung von Nukleinsäuren durch Vektoren, die eine DNA-Sequenz bestehend aus Antisense-Sequenzen der Nukleinsäuren, die identisch mit einer Nukleinsäure mit der Sequenz SEQ ID No. 1 sind, sowie eine für RNAse P kodierende Sequenz umfassen. Bei der Transkription solcher Vektoren entstehen in der Zelle RNA-Moleküle, die über eine Führungssequenz (die Antisense-Sequenz) verfügen, welche die RNAse P zur mRNA der Nukleinsäuren, die identisch mit einer Nukleinsäure mit der Sequenz SEQ ID No. 1 sind, führt, wodurch die Spaltung der mRNA durch RNAse P bewirkt wird (siehe auch US-Patent Nr. 5,168,053). Vorzugsweise umfasst die Führungssequenz 10 bis 15 Nukleotide, die zur DNA-Sequenz der Nukleinsäuren, die identisch mit einer Nukleinsäure mit der Sequenz SEQ ID No. 1 sind, komplementär sind.

Ein solches Verfahren kann z.B. die folgenden Schritte umfassen:
a) Herstellung eines Vektors, umfassend folgende Nukleinsäuresequenzen in 5'-3'-Orientierung:
   - eine in dem jeweiligen Mikroorganismus funktionelle Promotersequenz,
   - operativ daran gebunden eine Nukleinsäuresequenz, die zur Sequenz SEQ ID No.1 komplementär ist,
   - operativ daran gebunden eine DNA-Sequenz, die für Ribonuklease P kodiert,
   - operativ daran gebunden eine in dem jeweiligen Mirkoorganismus funktionelle Terminationssequenz; und
b) Übertragen des Vektors aus a) auf den Mikrooganismus und gegebenenfalls Integrieren des Vektors in dessen Genom.

Mit dem Begriff Komplementarität wird die Fähigkeit eines Nukleinsäuremoleküls beschrieben, mit einem anderen Nukleinsäuremolekül aufgrund von Wasserstoffbrücken zwischen komplementären Basen zu hybridisieren. Dem Fachmann ist bekannt, dass zwei Nukleinsäuremoleküle nicht über eine 100%ige Komplementarität verfügen müssen, um miteinander hybridisieren zu können. Vorzugsweise ist eine Nukleinsäure, die mit einer anderen Nukleinsäure hybridisieren soll, zu dieser zu mindestens 40%, bevorzugt zu mindestens 50%, ebenfalls bevorzugt zu mindestens 60% oder zu mindestens 70%, besonders bevorzugt zu mindestens 80%, ebenfalls besonders bevorzugt zu mindestens 90%, insbesondere bevorzugt zu mindestens 95% und am meisten bevorzugt zu mindestens 98% bzw. 100% komplementär.

Bevorzugt sind Komplementaritätsgrade wie Homologie und Identitätsgrade über die gesamte Protein- bzw. Nukleinsäurelänge zu bestimmen.

Stringente *in vitro*-Hybridisierungsbedingungen sind dem Fachmann bekannt und können der Literatur entnommen werden (siehe z.B. Sambrook et al., *vide supra).* Der Begriff spezifische Hybridisierung bezieht sich auf den Umstand, dass ein Molekül unter stringenten Bedingungen präferentiell an eine Nukleinsäure mit einer bestimmten Nukleinsäuresequenz bindet, wenn diese Nukleinsäure mit der bestimmten Nukleinsäuresequenz Teil einer komplexen Mischung von z.B. DNA- oder RNA-Molekülen ist.

Der Begriff stringente Bedingungen bezieht sich damit auf Bedingungen, unter denen eine Nukleinsäuren präferentiell an eine Nukleinsäure mit einer Zielsequenz bindet, aber nicht oder zumindest wesentlich reduziert an Nukleinsäuren mit anderen Sequenzen.

Stringente Bedingungen sind von den Umständen abhängig. Längere Sequenzen hybridisieren spezifisch bei höheren Temperaturen. Generell werden stringente Bedingungen so gewählt, dass die Hybridisierungstemperatur circa 5°C unter dem Schmelzpunkt (Tm) für die spezifische Sequenz bei einer definierten ionischen Stärke und einem definierten pH-Wert liegt. Tm ist die Temperatur (bei einem definierten pH-Wert, einer definierten ionischen Stärke und einer definierten Nukleinsäurekonzentration), bei der 50% der Moleküle, die zu einer Zielsequenz komplementär sind, mit dieser Zielsequenz hybridisieren. Typischerweise umfassen stringente Bedingungen Salzkonzentrationen zwischen 0,01 und 1,0 M Natriumionen (oder eines anderen Salzes) und/oder einen pH zwischen 7,0 und 8,3. Die Temperatur beträgt mindestens 30°C für kurze Moleküle, z.B. für solche, die zwischen 10 bis 50 Nukleotiden umfassen. Zusätzlich können stringente Bedingungen die Zugabe von destabilisierenden Agenzien wie z.B. Formamid umfassen.

Typische Hybridisierungs- und Waschpuffer haben folgende Zusammensetzung.

| *Prähybridisierungslösung:* | 0,5 % SDS | | |
|---|---|---|---|
| | 5 x SSC | | |
| | 50 mM NaPO₄, pH 6,8 | | |
| | 0,1% Na-Pyrophosphat | | |
| | 5 x Denhardt's Reagenz | | |
| | 100 µg/ml Lachssperm | | |
| | | | |

| *Hybridisierungslösung:* | Prähybridisierungslösung | | |
|---|---|---|---|
| | 1 x 10⁶ cpm/ml Sonde (5 bis 10 min 95°C) | | |
| | | | |

| *20 x SSC:* | 3 M NaCl | | |
|---|---|---|---|
| | 0,3 M Natriumcitrat | | |
| | ad pH 7 mit HCl | | |
| | | | |

| *50 x Denhardt's Reagenz:* | 5 g Ficoll | | |
|---|---|---|---|
| | 5 g Polyvinylpyrrolidon | | |
| | 5 g Bovine Serum Albumine | | |
| ad 500 ml A. dest. | | | |

| Eine Hybridisierung wird üblicherweise folgendermaßen durchgeführt: | | | |
|---|---|---|---|
| | | | |

| *Optional:* | Blot 30 min in 1 x SSC/ 0,1% SDS bei 65°C waschen | | |
|---|---|---|---|
| | | | |

| *Prähybridisierung:* | mindestens 2 h bei 50-55°C | | |
|---|---|---|---|
| | | | |

| *Hybridisierung:* | über Nacht bei 55-60°C | | |
|---|---|---|---|
| | | | |

| *Waschen:* | 5 min | 2 x SSC/ 0,1% SDS | Hybridisierungstemp. |
|---|---|---|---|
| | 30 min | 2 x SSC/ 0,1% SDS | Hybridisierungstemp. |
| | 30 min | 1 x SSC/ 0,1% SDS | Hybridisierungstemp. |
| | 45 min | 0,2 x SSC/0,1% SDS | 65°C |
| | 5 min | 0,1 x SSC | Raumtemperatur |

Die bei der Antisense-Strategie übertragenen Nukleinsäuren umfassen in der Regel zwischen 20 bis 1000 Nukleotide, bevorzugt zwischen 20 bis 750 Nukleotide, besonders bevorzugt um die 400 bis 800 und 500 bis 750 Nukleotide. Es können aber auch Nukleinsäuren verwendet werden, die zwischen 20 bis 500 Nukleotide, ebenfalls besonders bevorzugt zwischen 20 bis 300 Nukleotide, insbesondere bevorzugt zwischen 20 bis 150 Nukleotide, ebenfalls insbesondere bevorzugt zwischen 20 bis 75 Nukleotide und am meisten bevorzugt um die 20 bis 50 Nukleotide. Unter Umständen können die Sequenzen auch nur um die 20 oder 25 Nukleotide umfassen.

Wenn Nukleinsäuren auf die Mikroorganismen übertragen werden, deren Transkription in der Zelle zu Sequenzen führen, die zu mRNA der Nukleinsäure mit der Sequenz SEQ ID No. 1 komplementär sind (wie z.B. bei der antisense-Strategie), müssen diese Sequenzen nicht hundertprozentig komplementär zu der mRNA sein. Vielmehr reicht es aus, wenn diese Sequenzen zu mindestens 50%, bevorzugt zu mindestens 60%, besonders bevorzugt zu mindestens 70%, weiterhin besonders bevorzugt zu mindestens 80%, insbesondere bevorzugt zu mindestens 90% und am meisten bevorzugt zu mindestens 95% komplementär sind. Die Abweichungen können dabei durch Deletion, Substitution und/oder Insertion entstanden sein.

Generell gilt, dass nur solche komplementären Sequenzen erfindungsgemäß verwendet werden können, die in der Lage sind, spezifisch mit mRNA-Bereichen der Sequenz SEQ ID No. 1 zu hybridisieren. Sequenzen, die *in vivo* mit RNA-Bereichen von anderen Proteinen als NP_601931 bzw. dazu homologen Proteinen hybridisieren und deren Repression bewirken, sind für die erfindungsgemäßen Verfahren nicht geeignet.

Einige der oben erwähnten Verfahren können auch mit Sequenzen durchgeführt werden, die nicht Bestandteil des kodierenden Teils der mRNA von Nukleinsäuren mit der Sequenz SEQ ID No.1 sind oder dazu komplementär sind. Es kann z.B. ausreichend sein, wenn es sich um Sequenzen aus dem 5'- oder 3'-untranslatierten Bereich handelt, sofern diese regulatorischen Sequenzen charakteristisch für die mRNA der Nukleinsäure mit der Sequenz SEQ ID No. 1 bzw. der dazu homologen Sequenzen sind.

Solche Sequenzen können insbesondere dann eingesetzt werden, wenn die Translation einer mRNA durch antisense-Konstrukte inhibiert wird. Daher umfasst der Begriff mRNA erfindungsgemäß nicht nur die kodierenden Bestandteile der mRNA der Nukleinsäure mit der SEQ ID No. 1 bzw. der dazu homologen Sequenzen, sondern auch alle regulatorischen Sequenzen, die in der prä-mRNA oder reifen mRNA auftreten und die für die mRNA der Nukleinsäure mit der Sequenz SEQ ID No. 1 bzw. der dazu homologen Sequenzen charakteristisch sind. Dies gilt entsprechend auch für die DNA-Sequenz. Dabei kann es sich z.B. um 5'- und 3'-untranslatierte Bereiche handeln, um Promotorsequenzen, um upstream activating sequences etc.

Wenn Vektoren verwendet werden, deren Transkription zu RNA-Molekülen führt, die aus einer Führungssequenz und RNAse P bestehen, dann muss die Führungssequenz ausreichend komplementär sein, um spezifisch die mRNA der Nukleinsäure mit der Sequenz SEQ ID No. 1 bzw. der homologen Sequenzen zu erkennen. Welcher Bereich der mRNA durch die Führungssequenz erkannt wird, kann gemäß den jeweiligen Erfordernissen gewählt werden. Bevorzugt umfassen solche Führungssequenzen etwa 20 Nukleotide, sie sollten jedoch nicht wesentlich kürzer als 15 Nukleotide sein. Bei einer 100%-igen Komplementarität der Führungssequenz sollten auch zwölfNukleotide ausreichend sein. Selbstverständlich können die Führungssequenzen bis zu 100 Nukleotide oder mehr umfassen, da dadurch lediglich ihre Spezifität für die jeweilige mRNA erhöht wird.

Wenn im Rahmen der vorliegenden Erfindung von sense-Sequenzen gesprochen wird, dann sind damit diejenigen Sequenzen gemeint, die dem kodierenden Strang der Gene für NCg12640 bzw. dessen Homologen entsprechen oder Teile davon umfassen. Es handelt sich dabei um die Sequenz SEQ ID No. 1 bzw. deren funktionelle Homologe.

Wenn im Rahmen der vorliegenden Erfindung von antisense-Sequenzen gesprochen wird, dann sind damit diejenigen Sequenzen gemeint, die dem nicht kodierenden DNA-Strang der Gene für NP_601931 bzw. dessen Homologen entsprechen. Diese Sequenzen sind also komplementär zu SEQ ID No. 1 bzw. deren Homologen. Auch diese Sequenzen müssen selbstverständlich nicht hundertprozentig identisch mit der Sequenz des nicht-kodierenden DNA-Strangs sein, sondern können die vorstehend genannten Homologiegrade aufweisen. Dies kommt auch in dem Umstand zum Ausdruck, dass antisense-Sequenzen, die definitionsgemäß zu der mRNA eines Gens komplementär sind, nicht zu 100% komplementär zu dieser mRNA sein müssen. Sie können z.B. auch zu mindestens 50% komplementär, bevorzugt zu mindestens 60% komplementär, besonders bevorzugt zu mindestens 70% komplementär, weiterhin besonders bevorzugt zu mindestens 80% komplementär, insbesondere bevorzugt zu mindestens 90% komplementär und am meisten bevorzugt zu mindestens 95%, 98% und/oder 100% komplementär sein. Wie oben ausgeführt, ist es ausreichend, wenn die antisense-Sequenzen in der Lage sind, spezifisch mit der jeweiligen interessierenden mRNA von Nukleinsäuren mit der Sequenz SEQ ID No.1 zu hybridisieren. Die Hybridisierung kann entweder *in vivo* unter zellulären Bedingungen oder *in vitro* stattfinden.

Die Hybridisierung einer antisense-Sequenz mit einer endogenen mRNA-Sequenz findet typischerweise *in vivo* unter zellulären Bedingungen oder *in vitro* statt.

Die Begriffe sense und antisense sind darüber hinaus dem Fachmann bekannt. Dem mit der Genexpression vertrauten Fachmann ist entsprechend aus dem Stand der Technik auch bekannt, wie lang die zur Repression verwendeten Nukleinsäuremoleküle sein müssen und welche Homologie bzw. Komplementarität sie zu den jeweils interessierenden Sequenzen aufweisen müssen. Erfindungsgemäß können antisense-Sequenzen, die z.B. *in vivo* und/oder *in vitro* nicht spezifisch mit kodierenden sense-Sequenzen von NCg12640 oder dessen Homologen hybridisieren können, d.h. auch mit den kodierenden sense-Sequenzen von anderen Protein-Klassen hybridisieren, nicht verwendet werden.

Prinzipiell kann die antisense-Strategie mit einem Ribozym-Verfahren gekoppelt werden. Ribozyme sind katalytisch aktive RNA Sequenzen, die, gekoppelt an die antisense-Sequenzen, die Zielsequenzen katalytisch spalten (Tanner et al., (1999) FEMS Microbiol Rev. 23 (3), 257-75). Dies kann die Effizienz einer antisense-Strategie erhöhen.

Ferner ist eine Genrepression, aber auch die Genüberexpression, auch mit spezifischen DNA-bindenden Faktoren, z.B. Zinkfinger-Transkriptionsfaktoren möglich.

Des weiteren können Faktoren in eine Zelle eingebracht werden, die das Zielprotein selber inhibieren. Die proteinbindenden Faktoren können z.B. Aptamere sein (Famulok et al., (1999) Curr Top Microbiol Immunol. 243, 123-36).

Die Repression kann auch durch Aptamere erfolgen. Aptamere können so entworfen werden, dass sie spezifisch an NP_601931 binden und durch Kompetitionsreaktionen die Aktivität der Proteine reduzieren. Die Expression von Aptameren erfolgt üblicherweise durch Vektor-basierte Überexpression. Entwurf, Selektion und Expression von Aptameren sind dem Fachmann wohl bekannt (Famulok et al., (1999) Curr. Top. Microbiol. Immunol., 243,123-36).

Als weitere Protein-bindende Faktoren, deren Expression in Mikroorganismen eine Reduktion des Gehalts und/oder der Aktivität an den durch Nukleinsäuren, die identisch mit oder funktionell homolog zu einer Nukleinsäure mit der Sequenz SEQ ID No. 1 sind, kodierten Proteinen bewirkt, kommen für diese Proteine spezifische Antikörper in Frage. Die Herstellung solcher monoklonalen, polyklonalen oder rekombinanten Antikörper folgt Standardprotokollen (Guide to Protein Purification, Meth. Enzymol. 182, 663-679 (1990), M. P. Deutscher, ed.).

Die Expression von Antikörpern ist ebenfalls aus der Literatur bekannt (Fiedler et al., (1997) Immunotechnology 3, 205-216; Maynard and Georgiou (2000) Annu. Rev. Biomed. Eng. 2, 339-76).

Ein weiteres Verfahren zur Herstellung von Mikroorganismen sieht vor, dass die Aktivität der endogenen Nukleinsäuren, die identisch mit einer Nukleinsäure mit der Sequenz SEQ ID No. 1 sind, bzw. der dadurch kodierten Proteine dadurch verringert wird, dass in den Mikroorganismen nicht-funktionelle Mutanten dieser Nukleinsäuren bzw. Proteine exprimiert werden. Bei solchen nicht-funktionellen Mutanten bzw. Formen handelt es sich um Formen der Nukleinsäuren bzw. der durch die Nukleinsäure kodierten Proteine, die nicht mehr oder zumindest nur noch sehr beschränkt in der Lage sind, die Biosynthese von schwefelhaltigen Verbindungen und insbesondere Methionin in den Mikroorganismen zu regulieren. Solche nicht-funktionellen Mutanten können Punktmutationen, Insertionen und/oder Deletionen aufweisen. Sie sind insbesondere bei der Herstellung von Mikroorganismen nützlich, bei denen der Gehalt an endogenen Nukleinsäuren bzw. den durch diese Nukleinsäuresequenzen kodierten Proteine zwar nicht verändert wird, aber die Aktivität der endogenen Nukleinsäuren, die identisch mit einer Nukleinsäure mit der Sequenz SEQ ID No. 1 sind, bzw. der durch diese Nukleinsäuresequenzen kodierten Proteine durch Überexpression der vorstehend genannten nicht-funktionellen Mutanten blockiert wird.

Solche nicht-funktionellen Mutanten verfügen erfindungsgemäß über im Wesentlichen gleiche Nukleinsäure- bzw. Aminosäuresequenzen wie die Wildtyp-Formen, also z.B. die Sequenzen SEQ ID No. 1 und 2. Sie weisen jedoch an einigen Stellen Punktmutationen, Insertionen oder Deletionen von Nukleotiden oder Aminosäuren auf, die bewirken, dass die nicht-funktionellen Mutanten im Gegensatz zu den Wildtyp-Formen nicht oder nur sehr begrenzt in der Lage sind, mit ihren zellulären Bindungspartnern zu interagieren. Auf diese Weise kompetieren die nicht-funktionellen Mutanten die Interaktion der Wildtyp-Formen mit den zellulären Bindungspartnern aus und entkoppeln dadurch die Regulation der Biosynthese von schwefelhaltigen Verbindungen.

Solche funktionellen bzw. nicht-funktionellen Mutanten von Nukleinsäuren mit der Sequenz SEQ ID No.1 können vom Fachmann in einfacher Weise identifiziert werden. Dem Fachmann stehen eine Reihe von Techniken zur Verfügung, mit denen es möglich ist, Punktmutation(en), Insertion(en) oder Deletion(en) in die Nukleinsäuresequenzen, die zur SEQ ID No. 1 identisch oder dazu homolog sind, einzufügen (siehe u.a. Sambrook (2001), Molecular Cloning: A Laboratory Manual, 3. Auflage, Cold Spring Harbour Laboratory Press, Jäger et al., (1992) J. Bacteriol. 174, 5462-5465, WO 02/070685 sowie die sog. Quikchange Mutagenese-Methode von Stratagene, La Jolla, USA). Nach Einführung der Punktmutation, Insertion und/oder Deletion, die auch allgemein als Mutationen bezeichnet werden, kann der Fachmann durch entsprechende Tests, wie sie in den Beispielen dargestellt oder aus dem Stand der Technik bekannt sind, feststellen, ob die mutagenisierten Proteine noch über ihre normale Aktivität verfügen (siehe auch oben).

Erfindungsgemäß umfasst der Begriff "nicht-funktionelle Proteine bzw. Proteinfragmente" nicht solche Proteine, die keine wesentliche Sequenzhomologie auf Aminosäure- bzw. Nukleinsäureebene zu NCg12640 bzw. dessen Homologen aufweisen. Nicht-funktionelle Mutanten werden im Rahmen der Erfindung auch als inaktivierte oder inaktive oder dominant-negative Proteine bezeichnet.

Somit zeichnen sich die funktionellen und/oder nicht-funktionellen Mutanten, die die oben genannte Punktmutation(en), Insertion(en) und/oder Deletion(en) tragen, oder die funktionell äquivalenten Teile durch eine wesentliche Sequenzhomologie zu NCg12640 aus.

Die Frage, ob es sich bei einer nicht-funktionellen Mutante um eine Mutante im Sinne der vorliegenden Erfindung handelt, kann vom Fachmann in einfacher Weise ermittelt werden. Dazu wird die gewünschte Mutation, also Punktmutation, Insertion oder Deletion in die genomische Nukleinsäuresequenz, die zu der SEQ ID No. 1 identisch ist, z.B. durch homologe Rekombination oder ortsspezifische Mutagenes (site-directed mutagenesis) eingeführt und überprüft, ob durch diese Verringerung des Gehalts und/oder der Aktivität an endogenen Nukleinsäuresequenzen die Regulation der Biosynthese von schwefelhaltigen Verbindungen entkoppelt wird, so dass diese Mikroorganismen erhöhte Menge dieser schwefelhaltigen Verbindungen produzieren. Ist dies der Fall, ist damit gezeigt, dass es sich um eine nicht-funktionelle Mutation handelt.

Die so ermittelte nicht-funktionelle Mutation kann dann in eine DNA-Sequenz eingeführt werden, die sich in einem Vektor befindet und operativ verknüpft ist mit Promotor- und Terminationssequenzen, die in Mikroorganismen funktionell sind. Nach Überführen dieser Vektoren in die Mikroorganismen können die nicht-funktionellen Mutanten überexprimiert werden und kompetieren die Interaktion der endogenen Wildtyp-Sequenzen bzw. Proteine mit deren zellulären Bindungspartnern aus.

Ein solches Verfahren kann z.B. die folgenden Schritte umfassen:
a) Herstellen eines Vektors, umfassend folgende Nukleinsäuresequenzen in 5'-3'-Orientierung:
   - eine in Mikroorganismen funktionelle Promotersequenz,
   - operativ daran gebunden eine DNA-Sequenz, die für eine dominant-negative Mutante von NCg12640 bzw. ein Homologes davon, kodiert,
   - operativ daran gebunden eine in Mikroorganismen funktionelle Terminationssequenz,
b) Übertragen des Vektors aus a) auf den Mikroorganismus und gegebenenfalls Integrieren des Vektors in dessen Genom.

Dem Fachmann ist bekannt, wie Punktmutation(en), Insertionsmutation(en) oder Deletionsmutation(en) in die NP_601931bzw. dessen Homologe kodierenden Nukleinsäuresequenzen eingeführt werden können. PCR-Techniken können z.B. zur Einführung von Punktmutationen bevorzugt sein ("PCR technology: Principle and Applications for DNA Amplification", H. Ehrlich, id, Stockton Press). Beispiele zur Einführung von Punktmutationen in Nukleinsäuren mit der Sequenz SEQ ID No.1 finden sich zudem in den Ausführungsbeispielen.

Erfindungsgemäße Mikroorganismen können erfindungsgemäß auch derart hergestellt werden, dass z.B. ein rekombinanter Antikörper in den Mikroorganismen exprimiert wird, der spezifisch die Interaktion der durch die Nukleinsäuren, die identisch mit einer Nukleinsäure mit der Sequenz SEQ ID No. 1 sind, kodierten Proteine mit anderen zellulären Bindungspartnern kompetitiert.

Wie solche rekombinanten Antikörper gegen NP_601931 bzw. dessen Homologe isoliert und identifiziert werden können, ist dem Fachmann bekannt und kann der Literatur entnommen werden (Harlow et al., 1999, Using antibodies: a laboratory manual, Cold Spring Harbor Laboratory Press).

Unter rekombinanten Antikörpern werden erfindungsgemäß die bekannten unterschiedlichen Formen von rekombinanten Antikörpern verstanden, wie sie z.B. in Skerra et al. (Curr. Opin. Immunol. (1993) 2, 250 - 262) beschrieben wurden. Die erfindungsgemäßen rekombinanten Antikörper umfassen dabei die sogenannten Fab-Fragmente, Fv-Fragmente, scFv-Antikörper, scFv-Homodimere, die über Disulfit-Brücken miteinander verknüpft sind und sog. VH-Ketten. Die Fab-Fragmente bestehen aus assemblierten vollständigen leichten und verkürzten schweren Ketten, wohingegen Fv-Fragmente aus nicht-kovalent miteinander verknüpften VH- und VL-Ketten bestehen. Ein Überblick über die genannten Fragmente und rekombinanten Antikörper findet sich in Conrad et al. (Plant Mol. Biol. (1998) 38, 101 - 109). Die genannten Fab- und Fv-Fragmente können in vivo miteinander assoziieren.

Da dieser Prozess unter Umständen jedoch nicht sehr effizient abläuft, werden erfindungsgemäß bevorzugt scFv-Antikörper eingesetzt. Diese bestehen aus dem variablen Anteil der leichten Kette und dem variablen Anteil der schweren Kette, die über ein flexibles Linkerpeptid miteinander fusioniert sind. Die Herstellung solcher scFv-Antikörper ist im Stand der Technik intensiv beschrieben worden (siehe u.a. Conrad et al., vide supra; Breitling et al. (1999) Recombinant Antibodies, John Wiley & Sons, New York). Die scFv-Antikörper weisen die gleiche Antigen-Spezifität und Aktivität wie normale Antikörper auf, müssen aber nicht wie andere natürliche oder rekombinante Antikörper in vivo aus Einzelketten assembliert werden. Sie eignen sich deshalb insbesondere für die erfindungsgemäßen Verfahren.

In den oben angegebenen Referenzen wird ausführlich dargestellt, wie Nukleinsäuresequenzen, die für die erfindungsgemäß bevorzugten scFv-Antikörper kodieren, durch den Fachmann isoliert und hergestellt werden können.

Üblicherweise wird dabei von bestehenden Hybridoma-Zelllinien ausgegangen, die monoklonale Antikörper produzieren. Danach werden die für die leichte und schwere Ketten des Antikörpers kodierenden cDNAs isoliert und in einem zweiten Schritt die kodierenden Regionen für die variable Region der leichten und der schweren Kette in einem Molekül miteinander fusioniert.

Ein weiterer, dem Fachmann bekannter Weg zur Erzeugung rekombinanter Antikörper ist das Screening von Bibliotheken rekombinanter Antikörper (sogenannte "phage display libraries", siehe auch Hoogenboom et al. (2000) Immunology Today 21, 371-378; Winter et al. (1994) Annu. Rev. Immunol. 12,433-455; De Wildt et al. (2000) Nat. Biotechnol. 18, 989-994). Durch dem Fachmann bekannte Vorgehensweisen können bei diesem Verfahren rekombinante Antikörper gegen ein gegebenes Antigen angereichert, selektiert und isoliert werden.

Ein Verfahren zur Expression von Antikörpern gegen Proteine, die identisch mit oder homolog zu Proteinen mit der Sequenz SEQ ID No. 2 sind, kann z.B. die folgenden Schritte umfassen:
a) Herstellen eines Vektors, umfassend folgende Nukleinsäuresequenzen in 5'-3'-Orientierung:
   - eine in Mikroorganismen funktionelle Promotersequenz,
   - operativ daran gebunden eine DNA-Sequenz, die für einen rekombinanten Antikörper kodiert, der für Proteine, die identisch mit oder homolog zu Proteinen mit der Sequenz SEQ ID No. 2 sind, spezifisch ist,
   - operativ daran gebunden eine in Mikroorganismen funktionelle Terminationssequenz; und
b) Übertragen des Vektors aus a) auf den Mikroorganismus und gegebenenfalls Integrieren des Vektors in dessen Genom.

Wenn im Rahmen der vorliegenden Erfindung davon gesprochen wird, dass der Gehalt der Nukleinsäuren, die identisch mit zu einer Nukleinsäure mit der Sequenz SEQ ID No. 1 sind, bzw. der durch diese Sequenzen kodierten Proteine im Vergleich zum Wildtyp reduziert wird, wird dies ebenfalls dadurch beschrieben, dass die funktionelle Expression der Nukleinsäuren reduziert wird und bevorzugt vollständig unterbunden wird.

Im Rahmen der vorliegenden Erfindung umfasst der Begriff schwefelhaltige Verbindung bzw. Feinchemikalie jede chemische Verbindung, die wenigstens ein kovalent gebundenes Schwefelatom enthält und durch ein erfindungsgemäßes Fermentationsverfahrens zugänglich ist.

Erfindungsgemäß umfassen die schwefelhaltigen Verbindungen u.a. L-Methionin, L-Cystein, L-Homocystein, L-Cystathionin, S-Adenosyl-L-Methionin, Glutathion, Biotin, Thiamin und/oder Liponsäure, und vorzugsweise L-Methionin und/oder L-Cystein.

Erfindungsgemäß handelt es sich bei den Mikroorganismen um Corynebakterien. Besonders bevorzugt ist *Corynebacterium glutamicum.*

Der Begriff Stoffwechselmetabolit bezeichnet chemische Verbindungen, die im Stoffwechsel von Organismen als Zwischen- oder auch Endprodukte vorkommen und die neben ihrer Eigenschaft als chemische Bausteine auch modulierende Wirkung auf Enzyme und ihre katalytische Aktivität haben können. Dabei ist aus der Literatur bekannt, dass solche Stoffwechselmetabolite sowohl hemmend als auch stimulierend auf die Aktivität von Enzymen wirken können (Stryer, Biochemistry, (1995) W.H. Freeman & Company, New York, New York). In der Literatur ist auch beschrieben, dass es möglich ist, durch Maßnahmen wie Mutation der genomischen DNA durch UV-Strahlung, ionisierender Strahlung oder mutagene Substanzen und nachfolgender Selektion auf bestimmte Phänotypen in Organismen solche Enzyme zu produzieren, in denen die Beeinflussung durch Stoffwechselmetabolite verändert wurde (Sahm et al. (2000), Biological Chemistry 381(9-10): 899-910). Diese veränderten Eigenschaften können auch durch gezielte Maßnahmen erreicht werden. Dabei ist dem Fachmann bekannt, in Genen für Enzyme auch gezielt bestimmte Nukleotide der für das Protein kodierenden DNA so zu verändern, dass das aus der exprimierten DNA-Sequenz resultierende Protein bestimmte neue Eigenschaften aufweist, so zum Beispiel, dass die modulierende Wirkung von Stoffwechselmetaboliten gegenüber dem nicht veränderten Protein verändert ist.

Als erfindungsgemäße Mikroorganismen eignen sich insbesondere coryneforme Bakterien, die einen im Vergleich zum Wildtyp erniedrigten Gehalt an Nukleinsäuren, die identisch mit einer Nukleinsäure mit der Sequenz SEQ ID No. 1 sind, bzw. an durch diese Nukleinsäuren kodierten Proteine aufweisen.

Diese Mikroorganismen können schwefelhaltige Feinchemikalien, insbesondere L-Methionin aus Glucose, Saccharose, Lactose, Fructose, Maltose, Melasse, Stärke, Cellulose oder aus Glycerin und Ethanol herstellen. Aus der Gattung *Corynebacterium* ist insbesondere die Art *Corynebacterium glutamicum* geeignet, die für ihre Fähigkeit bekannt ist, L-Aminosäuren zu produzieren.

Beispiele für geeignete Stämme coryneformer Bakterien sind solche der Gattung
*Corynebacterium,* insbesondere der Art *Corynebacterium glutamicum*
*(C. glutamicum),* wie *Corynebacterium glutamicum* ATCC 13032,
*Corynebacterium acetoglutamicum* ATCC 15806,
*Corynebacterium acetoacidophilum* ATCC 13870,
*Corynebacterium thermoaminogenes* FERM BP-1539, und
*Corynebacterium melassecola* ATCC 17965,

oder der Gattung *Brevibacterium,* wie
*Brevibacterium flavum* ATCC 14067,
*Brevibacterium lactofermentum* ATCC 13869, und
*Brevibacterium divaricatum* ATCC 14020;

oder davon abgeleitete Stämme, wie
*Corynebacterium glutamicum* KFCC10065 und
*Corynebacterium glutamicum* ATCC21608,
welche ebenfalls die gewünschte Feinchemikalie oder deren Vorstufe(n) produzieren.

Die Abkürzung KFCC bezeichnet die *Korean Federation of Culture Collection,* die Abkürzung ATCC die *American Type Strain Culture Collection.*

Eine weitere erfindungsgemäße Ausführungsform betrifft Mikroorganismen, bei denen zusätzlich zu der Reduzierung des Gehalts der Nukleinsäuren, die identisch mit einer Nukleinsäure mit der Sequenz SEQ ID No. 1 sind, bzw. der durch diese Sequenzen kodierten Proteine der Gehalt von Nukleinsäuren, die identisch mit einer Nukleinsäure mit der Sequenz SEQ ID No. 3 sind, bzw. von durch diese letzteren Nukleinsäuren kodierten Proteinen im Vergleich zum Wildtyp reduziert ist.

Bei der Nukleinsäure mit der Sequenz SEQ ID No. 3 handelt es sich um eine Nukleinsäure, die für das Protein McbR mit der Aminosäuresequenz SEQ ID No. 4 aus *C. glutamicum* kodiert. Für dieses Protein konnte gezeigt werden, dass es ein transkriptioneller Repressor für Gene der Biosynthesewege von schwefelhaltigen Verbindungen und insbesondere Methionin in *C. glutamicum* ist (Rey et al., *vide supra).*

Durch die gleichzeitige Reduktion des Gehalts von Nukleinsäuren, die identisch mit Nukleinsäuren mit den Sequenzen SEQ ID No. 1 und 3 sind, werden Mikroorganismen bereitgestellt, die in besonderem Maße zur Herstellung von schwefelhaltigen Verbindungen geeignet sind. Bei den schwefelhaltigen Verbindungen kann es sich um die vorstehend genannten Verbindungen handeln, wobei Methionin und Cystein besonders bevorzugt sind.

Zur Erniedrigung des Gehalts von Nukleinsäuren, die identisch mit Nukleinsäuren mit der Sequenz SEQ ID No. 3 sind, bzw. den durch diese Nukleinsäuren kodierten Proteine können die gleichen Verfahren und Vorgehensweisen eingesetzt werden, wie sie vorstehend für die Nukleinsäuren, die identisch mit einer Nukleinsäure mit der Sequenz SEQ ID No. 1 sind, bzw. für die durch letztere Nukleinsäuren kodierten Proteine beschrieben worden sind. Begriffe wie Homologie, Komplementarität, funktionelle Formen, dominant-negative Mutanten etc. sind dabei gleichermaßen zu verstehen.

Eine weitere Ausführungsform der erfindungsgemäßen Mikroorganismen betrifft Mikroorganismen, in denen neben der im Vergleich zum Wildtyp reduzierten Gehalt an Nukleinsäuren, die identisch mit einer Nukleinsäure mit der Sequenz SEQ ID No. 1 sind, bzw. an durch diese Nukleinsäuren kodierten Proteine der Gehalt und/oder die Aktivität mindestens einer weiteren Nukleinsäure, die für ein Gen des Biosynthesewegs der gewünschten schwefelhaltigen Verbindungen kodiert, im Vergleich zum Wildtyp erniedrigt sind.

Diese weiteren Nukleinsäuren werden vorzugsweise ausgewählt aus der Gruppe I umfassend:
- das für Homoserine-Kinase kodierende Gen thrB (EP 1 108 790; DNA-SEQ ID No. 3453),
- das für Threonin-Dehydratase kodierende Gen ilvA (EP 1 108 790; DNA-SEQ ID No. 2328),
- das für Threonin-Synthase kodierende Gen thrC (EP 108 790; DNA-SEQ ID No. 3486),
- das für Meso-Diaminopimelat-D-Dehydrogenase kodierende Gen ddh (EP 1 108 790; DNA-SEQ ID No. 3494),
- das für Phosphoenolpyruvat-Carboxykinase kodierende Gen pck (EP 1 108 790; DNA-SEQ ID No. 3157),
- das für Glucose-6-Phosphat-6-Isomerase kodierende Gen pgi (EP 1 108 790; DNA-SEQ ID No. 950),
- das für Pyruvat-Oxidase kodierende Gen poxB (EP 1 108 790; DNA-SEQ ID No. 2873),
- das für Dihydrodipicolinat-Synthase kodierende Gen dapA (EP 1 108 790; DNA-SEQ ID No. 3476),
- das für Dihydrodipicolinat-Reduktase kodierende Gen dapB (EP 1 108 790; DNA-SEQ ID No. 3477),
- das für Diaminopicolinat-Decarboxylase kodierende Gen lysA (EP 1 108 790 A2; DNA-SEQ ID No. 3451),
- das für Glycosyltransferase (Genbank accession number NP_600345) kodierende Gen (Genbank accession number NCg11072), und
- das für Laktatdehydrogenase (Genbank accession number NP_602107) kodierende Gen (Genbank accession number NCg12817).

Daneben können letztere erfindungsgemäße Organismen selbstverständlich auch bezüglich des Gehalts der Nukleinsäuren, die identisch mit einer Nukleinsäure mit der Sequenz SEQ ID No. 3 sind, bzw. des Gehalts und/oder der Aktivität der durch diese Nukleinsäuren kodierten Proteine im Vergleich zum Wildtyp reduziert sein.

Eine Reduzierung des Gehalts und/oder der Aktivität bezüglich der in der Gruppe I genannten Nukleinsäuresequenzen bzw. bezüglich der durch diese Sequenzen kodierten Proteine kann dabei in der gleichen Weise erreicht werden, wie dies oben z.B. für die SEQ ID No. 1 bis 4 beschrieben wurde. Dabei sind die Begriffe wie Homologie, dominant-negative Mutante etc. so wie oben definiert zu verstehen.

Alle bisher genannten Ausführungsformen der vorliegenden Erfindung betreffen Mikroorganismen, die zur Herstellung der oben genannten schwefelhaltigen Verbindungen verwendet werden können. Insbesondere bevorzugt sind dabei Mikroorganismen, die zur Herstellung von Methionin und/oder Cystein verwendet werden können, wobei es sich bei diesen Mikroorganismen um die vorstehend genannten handeln kann und *C. glutamicum* sind. Dabei können zur Verringerung des Gehalts und/oder der Aktivität der vorstehend genannten Nukleinsäuren die vorstehend genannten Verfahren angewendet werden.

Eine weitere Ausführungsform der vorliegenden Erfindung betrifft die vorstehend genannten Mikroorganismen, in denen zusätzlich der Gehalt und/oder die Aktivität für Nukleinsäuren, die für ein Genprodukt des Biosynthesewegs der herzustellenden schwefelhaltigen Verbindungen kodieren, im Vergleich zum Wildtyp erhöht sind. Als Stoffwechselwege kommen dabei insbesondere die Biosynthesewege der Schwefel-Assimilation, des Methionin-Stoffwechsels, des Trehalose-Stoffwechsels, des Pyruvat-Stoffwechsels, des Cystein-Stoffwechselwegs, der Aspartatsemialdehyd-Synthese, der Glykolyse, der Anaplerotik, des Pentose-Phosphatstoffwechsels, des Zitronensäure-Zyklus oder des Aminosäure-Exports in Frage.

Insofern betreffen weitere Ausführungsformen der vorliegenden Erfindung Mikroorganismen, in denen der Gehalt und/oder die Aktivität von Nukleinsäuren bzw. von Proteinen, die durch diese Nukleinsäuren kodiert werden, erhöht ist, wobei die Nukleinsäuren ausgewählt sind aus der Gruppe II, umfassend
- das für Aspartatkinase kodierende Gen lysC (EP 1 108 790 A2; DNA-SEQ ID No. 281),
- das für Aspartat-Semialdehyd-Dehydrogenase kodierende Gen asd (EP 1 108 790 A2; DNA-SEQ ID No. 282),
- das für Glycerinaldehyd-3-Phosphat-Dehydrogenase kodierende Gen gap (Eikmanns (1992) Journal ofBacteriology 174: 6076-6086),
- das für 3-Phosphoglycerat-Kinase kodierende Gen pgk (Eikmanns (1992), Journal of Bacteriology 174: 6076-6086),
- das für Pyruvat-Carboxylase kodierende Gen pyc (Eikmanns (1992), Journal of Bacteriology 174: 6076-6086),
- das für Triosephosphat-Isomerase kodierende Gen tpi (Eikmanns (1992), Journal of Bacteriology 174: 6076-6086),
- das für Homoserin-O-Acetyltransferase kodierende Gen metA (EP 1 108 790;DNA-SEQ ID No. 725),
- das für Cystahionin-gamma-Synthase kodierende Gen metB (EP 1 108 790; DNA-SEQ ID No. 3491),
- das für Cystahionin-gamma-Lyase kodierende Gen metC (EP 1 108 790; DNA-SEQ ID No. 3061),
- das für Serin-Hydroxymethyltransferase kodierende Gen glyA (EP 1 108 790; DNA-SEQ ID No. 1110),
- das für O-Acetylhomoserin-Sulfhydrylase kodierende Gen metY (EP 1 108 790; DNA-SEQ ID No. 726),
- das für Methylentetrahydrofolat-Reduktase kodierende Gen metF (EP 1 108 790; DNA-SEQ ID No. 2379),
- das für Phosphoserin-Aminotransferase kodierende Gen serC (EP 1 108 790; DNA-SEQ ID No. 928),
- eines für Phosphoserin-Phosphatase kodierende Gen serB (EP 1 108 790; DNA-SEQ ID No. 334, DNA-SEQ ID No. 467, DNA-SEQ ID No. 2767),
- das für Serinacetyl-Transferase kodierende Gen cysE (EP 1 108 790; DNA-SEQ ID No. 2818),
- das für Homoserin-Dehydrogenase kodierende Gen hom (EP 1 108 790; DNA-SEQ ID No. 1306), und
- das für Methionin-Synthase kodierende Gen metH (EP 1 108 790; DNA-SEQ ID No. 1663),
- das für Methionin-Synthase (Genbank accession number NP_600367) kodierende Gen metE (Genbank accession number NCgl1094),
- das für Cystein-Synthase (Genbank accession number NP_601760) kodierende Gen (Genbank accession number NCgl2473),
- das für Cystein-Synthase (Genbank accession number NP_601337) kodierende Gen (Genbank accession number NCgl2055),
- das für Sulfit-Reduktase(Genbank accession number NP_602008) kodierende Gen (Genbank accession number NCgl2718),
- das für Phosphoadenosin-Phosphosulfat-Reduktase (Genbank accession number NP_602007) kodierende Gen cysH (Genbank accession number NCgl2717),
- das für Sulfatadenylyltransferase-Subunit 1 (Genbank accession number NP_602005) kodierende Gen (Genbank accession number NCg12715),
- das für CysN-Sulfatadenylyltransferase-Subunit 2 (Genbank accession number NP_602006) kodierende Gen (Genbank accession number NCgl2716),
- das für Ferredoxin-NADP Reduktase (Genbank accession number NP_602009) kodierende Gen (Genbank accession number NCgl2719),
- das für Ferredoxin (Genbank accession number NP_602010) kodierende Gen (Genbank accession number NCgl2720),
- das für Glucose-6-Phosphat-Dehydrogenase (Genbank accession number NP_600790) kodierende Gen (Genbank accession number NCgl1514), und
- das für Fructose-1-6-Bisphosphatase (Genbank accession number NP_601294) kodierende Gen (Genbank accession number NCgl2014).

Vorzugsweise beträgt die in erfindungsgemäßen Mikroorganismen bewirkte Erhöhung des Gehalts und/oder der Aktivität der durch die Nukleinsäuren der Gruppe II kodierten Proteine mindestens 5%, bevorzugt mindestens 20%, ebenfalls bevorzugt mindestens 50%, besonders bevorzugt mindestens 100%, ebenfalls besonders bevorzugt mindestens den Faktor 5, insbesondere bevorzugt mindestens den Faktor 10, ebenfalls insbesondere bevorzugt mindestens den Faktor 50, noch bevorzugter mindestens den Faktor 100 und am meisten bevorzugt den Faktor 1000.

Die Erhöhung des Gehalts und/oder der Aktivität der durch die Nukleinsäuren der Gruppe II kodierten Proteine kann auf verschiedene Weise erreicht werden. Bevorzugt wird dies dadurch geschehen, dass diese Nukleinsäuresequenzen in Form von DNA-Sequenzen in einen Vektor eingeführt werden, der zusätzlich einen in Mikroorganismen funktionellen Promotor, eine in Mikroorganismen funktionelle ribosomale Bindungsstelle sowie eine in Mikroorganismen funktionelle Terminationssequenz enthält. Dieser Vektor wird dann auf die Mikroorganismen übertragen und je nach gewähltem Vektor entweder extrachromosomal mit den Mikroorganismen mitrepliziert oder ins Genom der Mikroorganismen integriert. Dabei können im Wesentlichen die gleichen Vektoren bzw. Plasmide verwendet werden, wie sie vorstehend für die Erniedrigung des Gehalts und/oder der Aktivität bestimmter Nukleinsäuren bzw. der dadurch kodierten Proteine beschrieben wurden. Das Gleiche gilt für die verwendeten Selektionsmarker.

Darüber hinaus kann eine Erhöhung des Gehalts und/oder der Aktivität der durch die Nukleinsäuren der Gruppe II kodierten Proteine auch dadurch erreicht werden, dass funktionelle Äquivalente oder Analoga bzw. Homologe dieser Nukleinsäuren in den Mikroorganismen exprimiert werden. Bezüglich der Begriffe Homologie, Komplementarität etc. wird auf oben verwiesen.

Funktionale Äquivalente oder Analoga der konkret offenbarten Polypeptide sind im Rahmen der vorliegenden Erfindung von diesen Polypetiden verschiedene Polypeptide, welche weiterhin die gewünschte biologische Aktivität, wie z.B. Substratspezifität, besitzen.

Unter funktionalen Äquivalenten versteht man erfindungsgemäß insbesondere Mutanten, welche in wenigstens einer der Sequenzpositionen eine andere als die konkret genannte Aminosäure aufweisen, aber trotzdem eine wie oben genannten biologische Aktivität besitzen. Funktionale Äquivalente umfassend somit die durch eine oder mehrere Aminosäure-Additionen, -Substitutionen, -Deletionen und/oder-Inversionen erhältlichen Mutanten, wobei die genannten Veränderungen in jeglicher Sequenzposition auftreten können, solange sie zu einer Mutante mit dem oben erwähnten Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Funktionale Äquivalente umfassen selbstverständlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

Funktionale Äquivalente umfassen ebenfalls Fragmente, vorzugsweise einzelne Domänen oder Sequenzmotive, der erfindungsgemäßen Polypeptide, welche z.B. die gewünschte biologische Funktion aufweisen.

Funktionale Äquivalente sind außerdem Fusionsproteine, welche eine der vorstehend genannten Polypeptidsequenzen oder davon abgeleitete Äquivalente und wenigstens eine weitere, davon funktionell verschiedene, heterologe Sequenz in funktioneller N- oder C-terminaler Verknüpfung (d.h. ohne gegenseitige wesentliche funktionelle Beeinträchtigung der Fusionsproteinteile) aufweisen. Beispiele für derartige heterologe Sequenzen sind Signalpeptide, Enzyme, Immunoglobuline, Oberflächenantigene, Rezeptoren oder Rezeptorliganden.

Erfindungsgemäße Äquivalente sind Homologe zu den konkret offenbarten Proteinen. Diese besitzen wenigstens 90%, 95% oder 99%, Homologie zu einer der konkret offenbarten Sequenzen.

Homologe der erfindungsgemäßen Proteine oder Polypeptide können durch Mutagenese erzeugt werden, z.B. durch Punktmutation oder Verkürzung des Proteins. Der Begriff Homologes, wie er hier verwendet wird, betrifft eine Variante des Proteins, die als Agonist oder Antagonist der Protein-Aktivität wirkt.

Homologe der erfindungsgemäßen Proteine können durch Screening kombinatorischer Banken von Mutanten, wie z.B. Verkürzungsmutanten, identifiziert werden. Beispielsweise kann eine variierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang al. (1983) Tetrahedron 39, 3; Itakura et al. (1984) Annu. Rev. Biochem. 53, 323; Itakura et al., (1984) Science 198, 1056; Ike et al. (1983) Nucleic Acids Res. 11, 477). Zusätzlich können Banken von Fragmenten des Protein-Codons verwendet werden, um eine variierte Population von Protein-Fragmenten zum Screening und zur anschließenden Selektion von Homologen eines erfindungsgemäßen Proteins zu erzeugen. Bei einer Ausführungsform kann eine Bank von kodierenden Sequenzfragmenten durch Behandeln eines doppelsträngigen PCR-Fragmentes einer kodierenden Sequenz mit einer Nuklease unter Bedingungen, unter denen ein Nicking nur etwa einmal pro Molekül erfolgt, Denaturieren der doppelsträngigen DNA, Renaturieren der DNA unter Bildung doppelsträngiger DNA, die Sense-/Antisense-Paare von verschiedenen genickten Produkten umfassen kann, Entfernen einzelsträngiger Abschnitte aus neu gebildeten Dupleces durch Behandlung mit S1-Nuclease und Ligieren der resultierenden Fragmentbank in einen Expressionsvektor erzeugt werden. Durch dieses Verfahren kann eine Expressionsbank hergeleitet werden, die N-terminale, C-terminale und interne Fragmente mit verschiedenen Größen des erfindungsgemäßen Proteins kodiert.

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese von erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorbank und Exprimieren der kombinatorischen Gene unter Bedingungen, unter denen der Nachweis der gewünschten Aktivität und die Isolation des Vektors, der das Gen kodiert, dessen Produkt nachgewiesen wurde, erleichtert ist. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin t al. (1992) PNAS 89, 7811-7815; Delgrave et al. (1993) Protein Engineering 6(3), 327-331).

Die z.B. für das Enzym Methionin-Synthase (EC 2.1.1.13) kodierenden metH-Gene aus den Organismen obiger Gruppe II sind in an sich bekannter Weise isolierbar.

Zur Isolierung der metH-Gene oder auch anderer Gene der Organismen aus obiger Gruppe II wird zunächst eine Genbank dieses Organismus in *E. coli* angelegt. Das Anlegen von Genbanken ist in allgemein bekannten Lehrbüchern und Handbüchern ausführlich beschrieben. Als Beispiel seien das Lehrbuch von Winnacker: Gene und Klone, Eine Einführung in die Gentechnologie (Verlag Chemie, Weinheim, Deutschland, 1990), oder das Handbuch von Sambrook et al., *vide supra* genannt. Eine bekannte Genbank ist die des *E. coli* K-12 Stammes W3110, die von Kohara et al. (1987, Cell 50, 495-508) in λ-Vektoren angelegt wurde.

Zur Herstellung einer Genbank von Enzymen z.B. der Gruppe II in *E. coli* können Cosmide, wie der Cosmidvektor SuperCos I (Wahl et al., (1987) PNAS, 84, 2160-2164), aber auch Plasmide, wie pBR322 (BoliVal et al. (1979) Life Sciences, 25, 807-818 (1979)) oder pUC9 (Vieira et al. (1982), Gene, 19, 259-268) verwendet werden. Als Wirte eignen sich besonders solche E. coli-Stämme, die restriktions- und rekombinationsdefekt sind. Ein Beispiel hierfür ist der Stamm DH5αmcr, der von Grant et al. (1990) PNAS, 87, 4645-4649) beschrieben wurde. Die mit Hilfe von Cosmiden klonierten langen DNA-Fragmente können anschließend wiederum in gängige, für die Sequenzierung geeignete Vektoren subkloniert und anschließend sequenziert werden, so wie es z.B. bei Sanger et al. (1977, PNAS, 74, 5463-5467), beschrieben ist.

Die erhaltenen DNA-Sequenzen können dann mit bekannten Algorithmen bzw. Sequenzanalyse-Programmen, wie z.B. dem von Staden (1986, Nucleic Acids Research 14, 217-232), dem von Marck (1988, Nucleic Acids Research 16, 1829-1836) oder dem GCG-Programm von Butler (1998, Methods of Biochemical Analysis 39, 74-97), untersucht werden.

Anleitung zur Identifizierung von DNA-Sequenzen mittels Hybridisierung findet der Fachmann unter anderem im Handbuch "The DIG System Users Guide für Filter Hybridization" der Firma Boehringer Mannheim GmbH (Mannheim, Deutschland, 1993) und bei Liebl et al. (1991, International Journal of Sytematic Bacteriology, 41, 255-260). Anleitungen zur Amplifikation von DNA-Sequenzen mit Hilfe der Polymerase-Kettenreaktion (PCR) findet der Fachmann unter anderem im Handbuch von Gait: Oligonukleotide synthesis: A Practical Approach (IRL Press, Oxford, UK, 1984) und bei Newton und Graham: PCR (Spektrum Akademischer Verlag, Heidelberg, Deutschland, 1994).

Weiterhin ist bekannt, dass Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (1987, Journal of Bacteriology 169, 751-757), bei O'Regan et al. (1989, Gene 77, 237-251), bei Sahin-Toth et al. (1994, Protein Sciences 3, 240-247), bei Hochuli et al. (1988, Biotechnology 6, 1321-1325) und in bekannten Lehrbüchern der Genetik und Molekularbiologie.

Als Plasmide für alle vorstehend genannten Verfahren zur Erhöhung bzw. Erniedrigung des Gehalts und/oder der Aktivität von Nukleinsäuren bzw. dadurch kodierten Proteinen eignen sich solche, die in coryneformen Bakterien repliziert werden. Zahlreiche bekannte Plasmidvektoren, wie z.B. pZ1 Menkel et al. (1989, Applied and Environmental Microbiology 64, 549-554), pEKEx1 (Eikmanns et al., (1991, Gene, 102, 93-98) oder pHS2-1 (Sonnen et al. (1991, Gene, 107, 69-74)) beruhen auf den kryptischen Plasmiden pHM1519, pBL1 oder pGA1. Andere Plasmidvektoren, wie z.B. pCLiK5MCS, oder solche, die auf pCG4 (US-A 4,489,160) oder pNG2 (Serwold-David et al. (1990) FEMS Microbiology Letters 66, 119-124) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Reinscheid et al. (1994, Applied and Environmental Microbiology, 60, 126-132 zur Duplikation bzw. Amplifikation des hom-thrB-Operons beschrieben wurde. Bei dieser Methode wird das vollständige Gen in einen Plasmidvektor kloniert, der in einem Wirt (typischerweise *E.coli),* nicht aber in *C. glutamicum* replizieren kann. Als Vektoren kommen beispielsweise pSUP301 (Sirnon et al. (1983), Biotechnology 1, 784-791), pK18mob oder pK19mob (Schäfer et al., Gene 145, 69-73), Bemard et al. (1993, Journal of Molecular Biology, 234, 534-541)), pEM1 (Schrumpf et al. (1991), Journal of Bacteriology 173, 4510-4516) oder pBGS8 (Spratt et al. (1986), Gene 41, 337-342) in Frage. Der Plasmidvektor, der das zu amplifizierende Gen enthält, wird anschließend durch Transformation in den gewünschten Stamm von *C*. *glutamicum* überführt. Methoden zur Transformation sind beispielsweise bei Thierbach et al. (1988, Applied Microbiology and Biotechnology 29, 356-362)), Dunican et al (1989, Biotechnology 7, 1067-1070) und Tauch et al. (1994, FEMS Microbiological Letters 123, 343.347) beschrieben.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P.H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Selektionsmarker eignen sich die verschiedenen Antibiotika wie z.B. Chlorampicilin, Kanamycin, Gentamycin, G418, Bleomycin, Hygromycin, etc.

In einer bevorzugten Ausführungsform der vorliegenden Erfindung handelt es sich um Mikroorganismen, bei denen die Nukleinsäuren, die Proteine kodieren, deren Gehalt im Vergleich zum Wildtyp verringert werden soll, durch homologe Rekombination aus dem Genom der Mikroorganismen entfernt worden ist bzw. durch homologe Rekombination derart verändert sind, dass es zu keiner funktionellen Expression dieser Nukleinsäuresequenzen mehr kommt.

Diese erfindungsgemäß bevorzugten Mikroorganismen sind also hinsichtlich der genomischen Nukleinsäuren, die identisch mit. Nukleinsäuren mit den Sequenzen SEQ ID No. 1, SEQ ID No. 3 und den in der Gruppe I genannten Sequenzen sind, durch homologe Rekombination inaktiviert, so dass es zu keiner funktionellen Expression der durch die vorgenannten Nukleinsäure kodierten Proteine mehr kommt.

In einer weiteren bevorzugten Ausführungsform der vorliegenden Erfindung handelt es sich um Mikroorganismen, die zusätzlich Nukleinsäuren, die identisch mit bzw. homolog zu den Nukleinsäuren der Gruppe II sind, oder funktionelle Äquivalente davon überexprimieren.

Zur Erzielung einer Überexpression kann der Fachmann unterschiedliche Maßnahmen einzeln oder in Kombination ergreifen. So kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Methionin-Produktion zu steigern. Durch Maßnahmen zur Verlängerung der Lebensdauer der mRNA wird ebenfalls die Expression verbessert. Weiterhin wird durch Verhinderung des Abbaus des Enzyms ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (1987, Biontechnology 5, 137-146), bei Guerrero et al. (1994, Gene 138, 35-41), bei Tsuchiya et al. (1988, Biotechnology, 6, 428-430), bei Eikmanns et al. (1991, Gene, 102, 93-98), in EP 0 472 869, in US 4,601,893, bei Schwarzer et al. (1991, Biotechnology 9, 84-87), bei Remscheid et al. (1994, Applied and Environmental Microbiology, 60,126-132, bei LaBarre et al. (1993, Journal of Bacteriology 175, 1001-1007), in WO 96/15246, bei Malumbres et al. (1993, Gene 134, 15-24), in JP-A-10-229891, bei Jensen et al. (1998, Biotechnology and Bioengineering, 58,.191-195), bei Makrides (1996, Microbiological Reviews, 60, 512-538) und in bekannten Lehrbüchern der Genetik und Molekularbiologie.

Beispiele für Promotoren, die für im Rahmen der Erfindung angewendete Verfahren geeignet sind, umfassen die Promotoren groES, sod, eftu, ddh, amy, lysC, dapA, lysA aus *Corynebacterium glutamicum*, aber auch gram-positiven Promotoren SPO2 wie sie in Bacillus Subtilis and Its Closest Relatives, Sonenshein, Abraham L.,Hoch, James A., Losick, Richard; ASM Press, District of Columbia, Washington und Patek M., Eikmanns B. J., Patek J., Sahm H., Microbiology 1996,142 1297-1309 beschrieben sind, oder aber auch cos-, tac-, trp-, tet-, trp-tet-, lpp-, lac-, lpp-lac-, lacIq-, T7-, T5-, T3-, gal-, trc-, ara-, SP6-, 1-PR- oder im 1-PL-Promotor, die vorteilhafterweise in gram-negativen Bakterien Anwendung finden. Bevorzugt ist auch die Verwendung induzierbarer Promotoren, wie z.B. licht- und insbesondere temperaturinduzierbarer Promotoren, wie der PᵣPₗ-Promotor. Prinzipiell können alle natürlichen Promotoren mit ihren Regulationssequenzen verwendet werden. Darüber hinaus können auch synthetische Promotoren vorteilhaft verwendet werden. Weitere Beispiele für Promotoren sind in Patek M. et al., J. Biotechnol. 2003, 104, 311-323 beschrieben.

Die genannten regulatorischen Sequenzen sollen die gezielte Expression der Nukleinsäuresequenzen und, wo erwünscht, der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird. Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder Enhancer verwendet werden. Daneben ist aber auch eine Verstärkung der Translation möglich, indem beispielsweise die Stabilität der mRNA verbessert wird.

Dazu verwendet man gängige Rekombinations- und Klonierungstechniken, wie sie beispielsweise in Current Protocols in Molecular Biology, 1993, John Wiley & Sons, Incorporated, New York New York, PCR Methods, Gelfand, David H., Innis, Michael A., Sninsky, John J. 1999, Academic Press, Incorporated, California, San Diego, PCR Cloning Protocols, Methods in Molecular Biology Ser., Vol. 192, 2nd ed., Humana Press, New Jersey, Totowa. Sambrook et al., *vide supra* sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Die vorliegende Erfindung betrifft ebenfalls Verfahren zur Herstellung von schwefelhaltigen Verbindungen, bei denen es sich bevorzugt um die oben genannten schwefelhaltigen Verbindungen und insbesondere Methionin und/oder Cystein handelt. Diese Verfahren sind dadurch gekennzeichnet, dass sie Verwendung von den erfindungsgemäßen Mikroorganismen machen und die schwefelhaltigen Verbindungen vorzugsweise durch fermentative Anzucht dieser Mikroorganismen in den Zellen oder im Zellmedium angereichert werden. Dabei ist dem Fachmann bekannt, dass durch Optimierung der bevorzugten Fermentationsverfahren weitere Ausbeutesteigerungen erzielt werden können. In den erfindungsgemäßen Verfahren können die erfindungsgemäß hergestellten Mikroorganismen kontinuierlich oder diskontinuierlich im batch-Verfahren (Satzkultivierung) oder im fed batch-(Zulaufverfahren) oder repeated fed batch-Verfahren (repetitives Zulaufverfahren) zur Produktion von schwefelhaltige Verbindungen, insbesondere L-Methionin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker wie Mono-, Di- oder Polysaccharide. Besonders geeignete Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl, Sonnenblumenöl, Erdnussöl und Kokosfett, Fettsäuren wie z.B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten.

Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate und Sulfide, aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um Metallionen in Lösung zu halten. Besonders geeignete Chelatbildner umfassen Dihydroxyphenole wie Catechol oder Protocatechuat oder organische Säuren wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere L-Methionin enthaltenden, Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf ≥ 0 bis 3 g/l gehalten, beziehungsweise abgesenkt wird.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt bzw. aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich, die schwefelhaltigen Feinchemikalien, insbesondere L-Methionin, weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können ggf. wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die Identität und Reinheit der isolierten Verbindung(en) kann durch Techniken des Stands der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefasst in: Patek et al. (1994, Appl. Environ. Microbiol. 60, 133-140), Malakhova et al. (1996, Biotekhnologiya 11, 27-32), Schmidt et al. (1998, Bioprocess Engineer. 19, 67-70), Ulmann's Encyclopedia of Industrial Chemistry (1996, Bd. A27, VCH: Weinheim, S. 89-90, S. 521-540, S. 540-547, S. 559-566, 575-581 und S. 581-587); Michal, G (1999, Biochemical Pathways: An Atlas of Biochemistry and Molecular Biology, John Wiley and Sons), Fallon et al. (1987, Applications of HPLC in Biochemistry in: Laboratory Techniques in Biochemistry and Molecular Biology, Bd. 17).

Bevorzugt wird durch die erfindungsgemäßen Verfahren eine Steigerung der Herstellung von schwefelhaltigen Verbindungen, insbesondere von Methionin bzw. Cystein gegenüber dem Wildtyp um mindestens 5%, 10%, 50%, 75%, bevorzugt um mindestens den Faktor 2, 3, 4, 5, 7, 10, besonders bevorzugt mindestens um den Faktor 20, 40, 60, 80, 100, insbesondere bevorzugt um den Faktor 200, 500, 1000 und am meisten bevorzugt um den Faktor 10000 erreicht. In absoluten Mengen kann durch das erfindungsgemäße Verfahren insbesondere eine Ausbeute von 1 g bis 150 g Methionin pro Liter Fermentationsbrühe erzielt werden.

Die Erfindung wird nun anhand der folgenden, nicht limitierenden Beispiele näher beschrieben:

### Beispiele

### 1. Materialien und Methoden

### 1.1 Bakterienstämme, Medien und Plasmide

*C. glutamicum* ATCC 14752 oder ATCC 13032 wurden routinemäßig in CGXII-Minimalmedium (Keilhauer et al. (1993) J Bacteriol. 175, 5595-5603) kultiviert. Die Transposonmutanten wurden in Gegenwart von Kanamycin (15 mg/ml⁻¹) kultiviert. *E*. *coli* DH5α wurde für Standard-Klonierungen und *E.coli* ET12567 zur Plasmidamplifikation verwendet, wenn die Plasmide in *C*. *glutamicum* transformiert werden sollten.

Die in dieser Erfindung verwendeten Stämme und Plasmide sind in der Tabelle 1 dargestellt. Luria broth (LB) Medium, das mit entsprechenden Antibiotika (Kanamycin 50 µg ml⁻¹, Chloramphenicol, 50 µg ml⁻¹, Ampicillin, 100 µg ml⁻¹) versetzt war, wurde als Standardmedium für *E. coli-*Stämme verwendet. LB-Medium, das mit 4 mM MgSO₄ und 10 mM KCl (Psi-broth) versetzt war, wurde als "recovery"-Medium für chemisch transformierte *E*. *coli* verwendet. Das "recovery"-Medium für elektroporierte *C. glutamicum-*Stämme war LBHIS (Luria broth mit brain heart infusion) und Sorbitol (Liebl et al. (1989) FEMS Microbiol Lett, 65, 299-304). Das Plasmid pCGL0040 (*GenBank accession no.* U53587) wurde als Donor für das Transposon Tn*5531* (IS*1207* Km^{r}) verwendet und in *E. coli* ET12567 vervielfältigt.

### 1.2 Rekombinante DNA-Technologie

Die Transformation von E. coli-Zellen mit Plasmid-DNA wurde mit chemisch kompetentem *E. coli* DH5α oder ET12567 durchgeführt. Die Zellen wurden gemäß der Rubidium-Chlorid-Methode hergestellt [http://micro.nwfsc.noaa.gov/protocols/] und wie in Sambrook et al. *(vide supra*) transformiert. Die Herstellung von kompetenten *C*. *glutamicum-*Zellen und die Elektrotransformation wurde wie in der Literatur beschrieben durchgeführt (Ankri (1996), Plasmid, 35, 62 - 66; Liebl et al., *vide supra*).

Genomische DNA wurde unter Verwendung des Wizard^{®} genomic DNA-Purification Kit (Promega) isoliert. Plasmidpräparationen aus E. coli-Zellen wurden routinemäßig mit dem QIAprep Miniprep Kit (Qiagen) durchgeführt. Restriktionsendonukleasen wurden von Roche Diagnostics bezogen. Verdaute DNA-Fragmente wurden aus Agarosegel mit QIAEX II Gel Extraction Kit (Qiagen) gewonnen. Standard-DNA-Techniken wurden wie in der Literatur beschrieben durchgeführt (Sambrook et al., *vide supra*). DNA-Sequenzierungen wurden unter Verwendung des Global Edition IR2 System (LI-COR Inc., Lincoln, Nebr.) durchgeführt.

Als Genomdatenbank zur Identifizierung von ORFs und Promotoren wurde die ERGO-Datenbank (Integrated Genomics, Chicago, USA) verwendet. Die im Text angegebenen NCgl-Nummern beziehen sich auf die *C. glutamicum* ATCC 13032 Genomsequenz der Genbank am NCBI (Genbank accession no. NC 003450). Die aus der ERGO-Datenbank abgeleiteten Promotoren für die Cystein-Synthase (NCgl2473, Promotorposition 2721625-2721822), Sulfitreduktase (NCgl2718, Promotorposition 3005188-3005389) und O-Acetylhomoserinsulfhydrolase (*metY*, NCgl0625, Promotorposition 667771-668107) wurden mittels PCR amplifiziert und über *Xho*I und BamHI-Linker an Promotor-freies *lacZ* im Plasmid pClik (Cm^{r}) fusioniert, das durch die gleichen Restriktionsenzyme geöffnet worden war. Die erhaltenen Plasmide wurden als pClik-H185, pClik-H187 und pClik-H217 bezeichnet (siehe Tabelle 1). Eine einzelne BglII-Restriktionsschnittstelle in diesen Plasmiden wurde benutzt, um mittels PCR amplifiziertes NCgl2640 einzuführen, wofür das Primerpaar 2640-fwd-BglII (5'-CCGCTGCTGCTGGTGGCGCTAGATCTGCTAACGGC-3') und 2640-rev-BglII (5'-ATGTGTTGGGAGATCTCTTAAGTTATTTAGTCCAG-3') verwendet wurde. Das amplifizierte DNA-Fragment umfasste mutmaßliche regulatorische Elemente, die bis zu 370 Basenpaare stromaufwärts des NCgl2640-Gens lagen.

### 1.3 Transpose und Mutagenese, Screenen und Lokalisierung der Transpose- und Insertionsstellen

Das Plasmid pCGL0040 wurde aus E. *coli* ET12567 isoliert. Anschließend wurde *C. glutamicum* ATCC 14752 mit dem Plasmid mittels Elektroporation transformiert. Die Transposon-Insertionsmutanten wurden durch Ausplattieren auf LBHIS, das 20 µg Kanamycin ml-¹ enthielt, selektioniert. Alle erhaltenen Mutanten wurden vereinigt, zweimal mit sterilem 0,9 %-igem NaCl gewaschen und auf CGXII-Medium, das Kanamycin (20 µg ml⁻¹) und Ethionin (7,5 g l⁻¹) enthielt, in Form von 100 µl-Aliquots einer 10⁶-Verdünnung des vereinigten Pols ausplattiert. Der am schnellsten wachsende Klon wurde für die weitere Analyse ausgewählt.

Zur Lokalisation der Transposon-Insertionsstellen wurde genomische DNA aus den Mutanten isoliert. Die Insertionsstellen wurden dann durch Klonieren der Transposon-Chromosom-Verbindungsstellen in pUC18 und anschließende Sequenzierung mit dem Oligonukleotid Tn5531-Eco (5'-CGGGTCTACACCGCTAGCCCAGG-3') (Simic et al. (2001) J. Bacteriol. 183, 5317-5324) bestimmt. Die so erhaltenen Sequenzen wurden dann mittels des BLASTn-Programms gegenüber der NCBI-gene bank sequence und der ERGO-Datenbank analysiert. Verschiedene Sequenzanalysewerkzeuge (http://www.expasy.org/, http://npsa-pbil.ibcp.fr oder http://pfam.wustl.edu/ (Proteinfamiliendatenbank PFAM) wurden für Muster- und Profilsuchen mit der NCg12640-Sequenz eingesetzt.

### 1.4 Chromosomale Deletion von NCg12640 in C. glutamicum ATCC13032

Mittels der zwei Primerpaaren
2640-SacB 1 (5'-GAGAGGGCCCATCAGCAGAACCTGGAACC-3') / 2640-SacB2 (5'-GATCCAGAGGTCCACAACC-3') und
2640-SacB3 (5'-GATGGTTCAAGACGAACTCC-3') / 2640-SacB4
(5'-GAGAGTCGACCAGAATCAATTCCAGCCTTC-3')
wurden die stromaufwärts und stromabwärts liegende Region von NCg12640 durch PCR aus chromosomaler DNA von C. *glutamicum* ATCC 13032 amplifiziert. Die erhaltenen Fragmente wurde mit *Apa*I*lXba*I oder *Spe*I/*Sal*I verdaut und zusammen in einen nicht-replikativen Vektor pClik-SacB kloniert, der mit *Apa*I*lSal*I verdaut wurde, wodurch das Plasmid pSdel-NCgl2640 erhalten wurde. *C. glutamicum* ATCC13032 wurde dann mittels Elektroporation mit dem nicht-replikativen Plasmid pSdel-NCg12640 transformiert. Kanamycin-resistente Klone enthielten die chromosomal integrierten Plasmide. Anschließend wurde auf den Verlust des Plasmids selektioniert, indem nach Saccharose-resistenten Mutanten gemäß der Methode von Schäfer et al. gescreent wurde (Schäfer et al. (1994) Gene 145:69-73). Die Deletion wurde mittels PCR-Analyse und Southern-Blotting verifiziert.

### 1.5 LacZ-Aktivitätsmessungen

Ausgewählte mutierte Stämme von C. *glutamicum* wurden mit den pClik-Plasmiden H185,
H187, und H217 oder den mit NCg12640 komplementierten Derivaten davon (siehe Tabelle 1) komplementiert. Die Transformanten wurden in CGXII-Minimalmedium, das Kanamycin (20 µg ml⁻¹) und Chloramphenicol (15 µg ml⁻¹) enthielt, in der Gegenwart oder Abwesenheit von 10 mM L-Methionin angezogen. Zellen wurden bis zur frühen Log-Phase (OD₆₀₀ = 1 - 2) angezogen und hinsichtlich einer β-Gal-Aktivität wie in Sambrook et al. beschrieben (Sambrook et al., *vide supra*) untersucht. Die Assays wurden jeweils dreimal in vier unabhängigen Testserien durchgeführt.

### 1.6 Isolierung von DNA-bindenden Proteinen

Das Prinzip der Isolierung von DNA-bindenden Proteinen mittels DNA-Affinitätschromatographie unter Verwendung von magnetischen Beads ist im Wesentlichen von Gabielsen et al. (1993), Methods Enzymol., 218, 508-225) beschrieben worden und ein detailliertes Protokoll ist für C. *glutamicum* erhältlich, siehe Rey et al. *(vide supra*). Vorliegend wurde letzteres Protokoll mit einigen geringen Ausnahmen angewendet. Mit Ausnahme der Elutionspuffer wurden alle Puffer mit 2,5 mM L-Methionin versetzt. Unmittelbar nach der Zelldisruption, wurden die rohen Extrakte gegen Proteolyse durch ein Protease-Inhibitor (PMSF, Aprotinin, Leupeptin (Rosenberg et al. (1996) Protein Analysis and Purification. Benchtop Techniques, Boston, Birkhäuser*)* geschützt. Nach der Ultrazentrifugation des rohen Extrakts (200000 g, 40 min. 40°C) wurde die Proteinlösung durch Gelfiltration (Sephadex G25) entsalzt. Biotinylierte PCR-amplifizierte Promotor-DNA wurde auf Streptavidin-beschichteten Dynabeads® M270 (Dynal Biotech.) immobilisiert. Als ein negatives Kontrollfragment wurde ein 460 bp-Fragment aus der stromaufwärts liegenden Region des *groES*-Gens aus *C. glutamicum* amplifiziert. Die Waschpuffer enthielten hohe Mengen an unspezifischer Kompetitor-DNA (Lachssperma DNA, 0,4 mg ml⁻¹, Sigma).
I D-SDS-PAGE wurde mit einer 4%-igen Sammelgel und einem 12%-igen Trenngel (Schägger et al. (1987) Anal. Biochem. 166, 368-379) durchgeführt und die Proteine wurden mit kolloidalem Coomassie Brilliant Blue G-250 angefärbt. Das Protokoll für den tryptischen Verdau und die MALDI-TOF-Analyse entspricht im Wesentlichen dem in Hermann et al. (2001) Electrophoresis, 22, 1712-1723 beschriebenen Protokoll.

### 1.7 Bestimmung der extra- und intrazellulären Methionin-Konzentrationen

Methionin wurde in Form seines o-Phthaldialdehyd-Derivats durch HPLC (Molnar-Perl et al. (2001), J. Chromatogr. 913, 283-302) quantifiziert. C. *glutamicum* wurde bis zur stationären Phase in 500 ml-Schüttelbechern mit Kulturvolumen von 50-100 ml bei 30°C und 225 rpm angezogen. Die Zellen wurden mittels Zentrifugation (10.000 g, 10 min. 4°C) entfernt und die Methionin-Konzentration durch HPLC-Analyse bestimmt. Für die Bestimmung von intrazellulären Methionin-Konzentrationen wurden die Zellen von der Flüssigkeit abgetrennt und durch Siliziumöl-Zentrifugation aktiviert (Ebbighausen et al. (1989) Appl. Microbiol. Biotechnol., 31, 184-190). Anschließend wurden die Zellen durch Ultraschallbehandlung oder durch einen "blue-capped" Ribolyser (FastPrep®, Q-Biogene) aufgeschlossen. Das lösliche Protein im Überstand wurde nach einem Bradford-Assay bestimmt. Der intrazelluläre Gehalt an löslichem Protein in *C. glutamicum* wurde somit empirisch als 250 mg ml⁻¹ bestimmt. Basierend auf diesem Wert, wurde das komplette interne Zellvolumen einer Probe kalkuliert, um Methionin-Konzentration in Folge der HPLC-Analyse bestimmen zu können.

### 2. Ergebnisse

### 2.1 Selektion und Identifizierung von Ethionin-resistenten Transposon-Mutanten

Im Rahmen der vorliegenden Erfindung wurde von der Hypothese ausgegangen, dass eine Ethionin-Resistenz durch Überproduktion von Methionin erreicht werden kann, die wiederum durch Inaktivierung von putativen Repressoren, die in die Regulation der Methionin-Biosynthese in C. *glutamicum* involviert sein könnten, erreicht werden kann. Der Grund liegt darin, dass es sich bei Ethionin um ein Strukturanalogon von Methionin handelt, das nicht metabolisiert werden kann und somit hohe Konzentrationen an Methionin vorspiegelt. In Folge davon wird üblicherweise die Methionin-Biosynthese herunterreguliert und der Organismus stirbt schließlich an einem Mangel an Methionin.

Eine der Möglichkeiten, die ein Organismus einsetzen kann, um diese durch ein Antimetabolit verursachte Toxizität zu umgehen, ist den toxischen Wirkstoff auszukompetieren, indem der natürliche Metabolit, in diesem Fall Methionin, überproduziert wird. Auf diese Weise wurden bereits in den siebziger Jahren Methioninresistente Stämme hergestellt (Kase et al., *vide supra*). Allerdings konnten damals keine Stämme erhalten werden, die Methionin in signifikanter Weise überproduzieren, vermutlich deshalb, weil in den damals durchgeführten Selektionsexperimenten keine Mutanten identifiziert wurden, die als Zentralregulator der Biosynthese von Methionin deaktivieren. Dies war einer der Gründe, warum vorliegend versucht wurde, die Mutationen durch Transposon-vermittelte Mutagenese einzuführen.

Es wurde daher *C. glutamicum* ATCC 14752 mit pCGL0040 als Donor für das Transposon Tn5531 transformiert. Auf diese Weise wurden etwa 7000 Mutanten auf LBHIS-Platten mit Kanamycin erhalten. Diese Mutanten wurden wie im Stand der Technik beschrieben gepoolt und auf Platten, die CGXII-Medium mit 7,5 g D,L-Ethionin l⁻¹ plus Kanamycin enthielten, ausplattiert. Es wurden etwa 100.000 colony forming units (CFU) ausplattiert, um sicherzustellen, dass sämtliche möglicherweise Ethionin-resistenten Mutanten wiedergewonnen werden konnten. Das Wachstum des Wildtyps war bei 6 g l⁻¹ Ethionin für mindestens 4 Tage inhibiert. Nach 2 Tagen konnten 11 Kanamycin und Ethionin-resistente Mutanten isoliert werden. Alle Mutanten enthielten die identische Tn5531-Insertion in dem ORL NCg12640. Diese Mutation wurde als 14752-Δ2640 bezeichnet und ein Klon wurde für die weiteren Experimente ausgewählt (Tabelle 1).

### 2.2 Sequenzanalyse der Transposon-Insertionsstellen

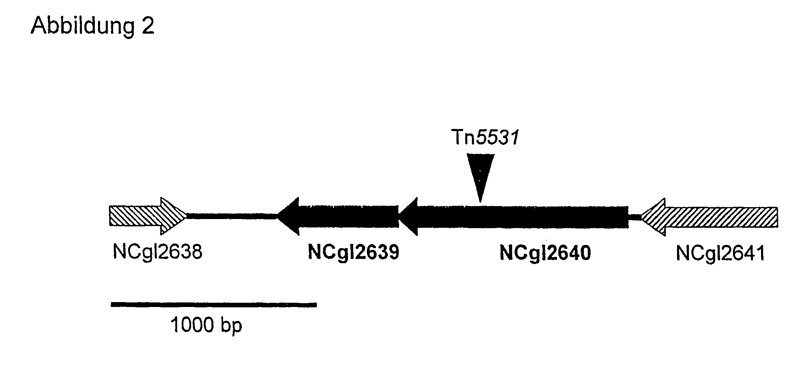

Es konnte festgestellt werden, dass die Stelle der Transposon-Insertion in Stamm 14752::2640 in der C-terminalen Hälfte des putativen Proteins in der Position 2918026/2918027 *(Genbank accession number* NC 003450) liegt. NCg12640 ist von NCgl2639 durch 7 Basenpaare getrennt, so dass beide Gene vermutlich als ein Operon organisiert sind. NCgl2539 ist in der Datenbank (Genbank) als eine putative Hydrolase oder Acetyltransferase annotiert (siehe Abbildung 2). NCg12640 kodiert für ein Protein von 42 kDa. Durch Homologie konnten mehr als 25 putative bakterielle Proteine von signifikanter Homologie (e-Wert < 2e-20) identifiziert werden, von denen keines bisher in funktioneller Weise annotiert worden ist. Durch eine Suche nach konservierten Domänen konnte ein Konsensus-Muster für Proteine unbekannter Funktion (COG2170) mit hoher Signifikanz identifiziert werden, ebenso wie für Motiv 04107 der Proteinfamilien-Datenbank (PFAM), das charakteristisch für die Glutamat-Cystein-Ligase-Familie ist. Weitere Konsensusmotive konnten nicht identifiziert werden. Insbesondere konnten keine DNA-bindenden Proteine identifiziert werden.

### 2.3 Verifizierung des Methionin-resistenten Phänotyps

*C. glutamicum* ATCC 14752 und der mutierte Stamm wurden dann in CGXII-Medium, das 3 g l⁻¹-Glukose enthielt, in Gegenwart oder Abwesenheit von 7,5 g l⁻¹ (Mutante) oder 3,8 g l⁻¹ (Wildtyp) D, L-Ethionin angezogen. Das Wachstum der Mutante in Gegenwart von Ethionin war weder vom Wachstum ohne Methionin noch vom Wachstum des Wildtyps ohne Ethionin unterscheidbar. Das Wachstum des Wildtyps in der Gegenwart wesentlich geringerer subletaler Konzentrationen von Ethionin war dagegen signifikant beeinträchtigt (siehe Abb. 3).

Um auszuschließen, dass Mutationen an anderen Stellen den Ethionin-resistenten Phänotyp verursacht haben könnten, wurde der Effekt einer Inaktivierung von NCgl2640 in *C*. *glutamicum* ATCC 13032 getestet. NCg12640 wurde durch homologe Rekombination und die Selektion von Sucrose-toleranten Mutantenstämmen aus dem Genom von *C*. *glutanicum* ausgeschnitten. Diese Mutante, die als 13032::2640 bezeichnet wurde, war gegenüber D, L-Ethionin bei 7,5 g l⁻¹ resistent, wohingegen kein Wachstum des Wildtyps ATCC 13032 auf diesen Platten detektiert werden konnte.

Damit konnte im Rahmen der vorliegenden Erfindung nachgewiesen werden, dass der Ethionin-resistente Phänotyp in der Tat ausschließlich auf der Inaktivierung von NCg12640 beruht.

### 2.4 Veränderte Expression von Methionin-Biosynthese-Genen in der NCgl2640-Mutante

Es wurde dann untersucht, ob in der NCgl2640-Mutante die normalerweise strikte Regulation des Schwefeleinbaus, der einen Schlüsselschritt der Methionin-Biosynthese darstellt, verändert ist.

Es wurde von der Hypothese ausgegangen, dass, wenn es sich bei NCg12640 um einen zentralen Regulator der Biosynthese von Schwefelverbindungen in *C. glutanicum* handelt, dieser die Expression von Genen, die in die Assimilation von Schwefel involviert sind, reguliert und es zur Expression von erhöhten Methionin-Mengen kommen müsste, die für die Ethionin-Resistenz verantwortlich wären. Diese Hypothese wurde anhand des Einflusses eines NCgl2640 Knock-outs auf die Expressionslevels von *metY*, *cysK* und Sulfatoperongenen untersucht. Dafür wurden der Stamm 14752::2640, der bezüglich NCg12640 deletiert ist, und der Wildtyp mit *lacZ*-Reporterplasmiden pClik-PcysK, pClik-PmetY und pClik-Psulfat (siehe auch Tab. 1) transformiert. Der ATCC 14752-Wildtyp und der mutierte Stamm wurden dann bis zur Log-Phase (OD₆₀₀ = 3) in CGXII-Medium mit oder ohne Zugabe von 10 mM L-Methionin angezogen und die Genexpression mittels der *lacZ*-Aktivität bestimmt.

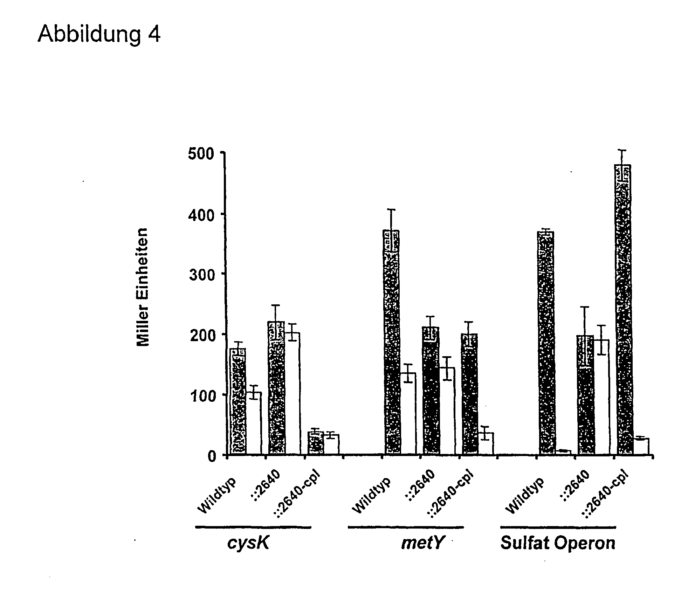

Im Wildtyp reduzierte das Vorhandensein von Methionin die Expressionslevel aller untersuchten Gene. Die Expression des Sulfatoperons war komplett unterdrückt. In dem mutierten Stamm wurde eine signifikante Derepression für *cysK* und das Sulfatoperon beobachtet. Bei beiden Genen war die Expression von Methionin unabhängig, was eine komplette Derepression, d.h. eine komplette Deregulation des Biosyntheseweges für Methionin und andere Schwefel-haltige Verbindungen in dem mutierten Stammhintergrund andeutet (siehe auch Abb. 4). Um polare Effekte der Inaktivierung von NCg12640 auf das daneben liegende NCgl2639 (Hydrolase- oder Acetyltransferase, siehe Abb. 2) auszuschließen, wurde NCg12640 über seinen Promotor in der Mutante exprimiert, was den Wildtyp-Phänotyp wieder herstellte, d.h. eine Methionin-induzierte Repression und geringere (*cysK*) oder höhere (Sulfatoperon) basale Transkriptionslevel (siehe auch Abb. 4) wurden beobachtet. Dieser Phänotyp ist in den komplementierten Stämmen sogar noch stärker ausgebildet als im Wildtyp. Der Grund liegt vermutlich darin, dass im komplementierten Stamm die Expression von NCgl2640, die von einem "medium copy"-Plasmid aus erfolgte, erhöht ist.

### 2.5 NCg12640 bindet nicht an die Promotoren von cysK, metY und Sulfatoperons

Im Rahmen der vorliegenden Erfindung konnte somit gezeigt werden, dass die Expression von *cysK*, dem Sulfatoperon und auch von *metY* durch NCg12640 in zentraler Weise reguliert wird. Da klassische DNA-Bindungsmotive nicht in der Sequenz von NCg12640 identifiziert werden konnten, wurde eine DNA-Affinitätsreinigung in einem so genannten "Pull-Down-Assay" angewendet, um zu überprüfen, ob NCgl an die jeweiligen Promotorregionen der oben genannten Gene binden kann. Die durch PCR amplifizierten und an Beads immobilisierten Promotoren wurden in Gegenwart von 2,5 mM L-Methionin mit rohen Extrakten von *C. glutanicum-*Zellen, die in Gegenwart oder Abwesenheit von 10 mM L-Methionin kultiviert worden waren, inkubiert. Proteine, die von den Promotoren bei Hochsalzkonzentration (> 200 mM) eluierten, wurden durch 1 D-SDS-PAGE aufgetrennt und mittels MALDI-TOF analysiert.

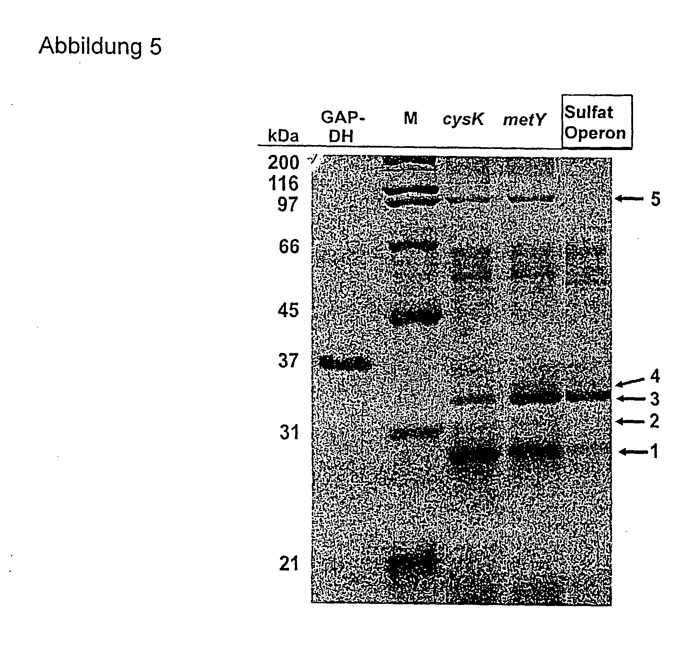

Mit einem ähnlichen Ansatz hatten Rey et al. *(vide supra*) zuvor den McbR-Repressor zusammen mit vier weiteren Proteinen identifiziert, die spezifisch den *metY-*Promotor zu binden scheinen. Im Rahmen der vorliegenden Erfindung konnten diese Ergebnisse bestätigt werden. Darüber hinaus konnte gezeigt werden, dass McbR ebenfalls an die Promotoren von *cysK* und das Sulfatoperon bindet (siehe Abb. 5). In beiden Studien konnte jedoch NCg12640 nicht nachgewiesen werden, was darauf hindeutet, dass eine direkte DNA-Protein-Interaktion nicht an der durch NCgl2640 vermittelten Regulation beteiligt ist.

### 2.6 Erhöhte L-Methionin-Mengen in der NCgl2640-Mutante

Die Resistenz von C. *glutanicum* 14752::2640 gegenüber hohen Mengen an Ethionin scheint somit eine Folge einer erhöhten Biosynthese von L-Methionin zu sein. Um diese Hypothese zu bestätigen, wurde die Herstellung von L-Methionin im Wildtyp und in Mutanten in CGXII Minimalmedium "batch"-Kulturen überprüft.

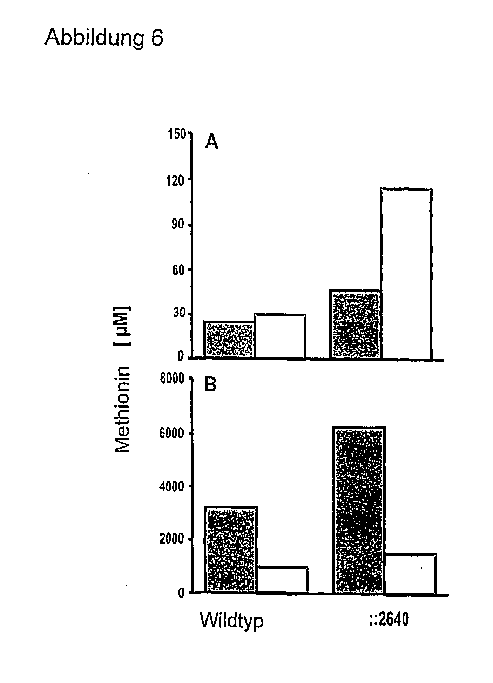

Es konnte gezeigt werden, dass der mutierte Stamm in der Regel mindestens zweimal soviel Methionin wie der Wildtyp produzierte. In Gegenwart von Ethionin konnte die Methionin-Sekretion in der Mutante, aber nicht im Wildtyp stimuliert werden (siehe Abb. 6A). Intrazelluläre Methionin-Mengen waren in gleicher Weise in der Mutante verdoppelt, wohingegen durch Ethionin die intrazelluläre Methionin-Menge vermindert wurde, was vermutlich auf eine Stimulation der Methionin-Sekretion beruht (siehe Abb. 6B). Die gesamte Methionin-Menge war in der Mutante doppelt so hoch wie im Wildtyp.

Die oben dargestellten Experimente zeigen, dass Mikroorganismen, in denen die kodierenden Sequenzen für NCg12640 deletiert sind und somit der Gehalt und/oder die Aktivität an durch diese Nukleinsäure kodiertem Protein im Vergleich zum Wildtyp vermindert sind, in der Lage sind, Methionin in erhöhten Mengen zu produzieren.

Da durch NCg12640, bei dem es sich wahrscheinlich um einen trans-agierenden Regulator handelt, die Gene des Schwefel-Stoffwechsels reguliert werden, können die erfindungsgemäßen Mikroorganismen auch zur Herstellung anderer schwefelhaltiger Verbindungen verwendet werden. Dies ist darin begründet, dass z.B. auch zur Herstellung von Cystein seitens der Mikroorganismen auf die Stoffwechselwege, die zur Reduktion des Schwefel führen, zurückgegriffen werden muss und weitere schwefelhaltige Verbindungen wie z.B. Glutathion oder S-Adenosylmethionin vom Vorhandensein von Cystein und/oder Methioin abhängen.

### Sequenzdaten

SEQ ID No.1 (Genbank accession number NCgl2640):

SEQ ID No.2 (Genbank accession number NP_601931):

SEQ ID No.3 (McbR):

SEQ ID No.4 (Genbank accession number NP_602128):

### Abbildungslegenden

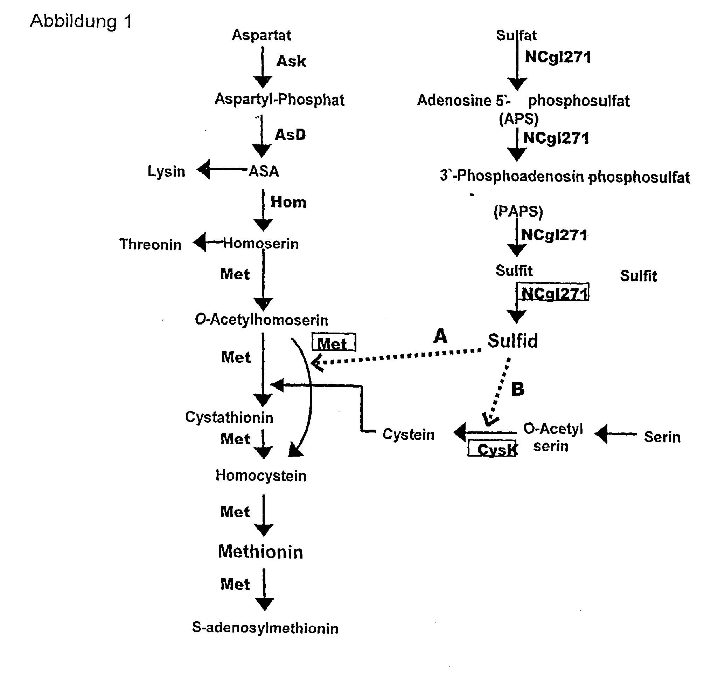

### Abb. 1 Der getrennte Weg zur Inkorporation von Schwefel in der Methioninbiosynthese

Schwefel kann entweder über direkte Sulfhydrolation, die durch MetY (A) katalysiert wird, oder durch den Transsulfhydrolationsweg (B) inkorporiert werden. *lacZ*-Fusionen wurden für Promotoren der markierten Genprodukte hergestellt. Der Promotor von NCgl2718 steuert die Expression von Genen, die im Sulfat-Cluster (NCgl2715-NCgl2720) organisiert sind.

Folgende Abkürzungen wurden verwendet: Ask, Aspartatkinase, AsDH, Aspartatsemialdehyd-Dehydrogenase, Hom, Homoserin-Dehydrogenase, MetA, Homoserin-Acetyltransferase, MetB, Cystathionin-γ-Synthase, MetC, Cystathionin-β-Lyase, MetH, Methioninsynthase, MetY, O-Acetylhomoserin-Sulfhydrolase, MetK, S-Adenosylmethionin-Synthase, NCgl2715, Sulfat-Adenosyltransferase Untereinheit 1; NCgl2716, Sulfat-Adenosyltransferase Untereinheit 2; NCgl2717, PAPS-Reduktase; NCgl2718, Sulfidreduktase, die als putative Nitritreduktase annotiert ist; CysK, Cystein-Synthase.

### Abb. 2 Der genomische Kontext von NCgl2640

Die Genbank-Annotationen, die für die ORFs erhältlich sind:
NCgl2638, Ähnlichkeiten zu einem vielteiligen Na⁺/H⁺-Antiporter;
NCgl2639, ähnlich zu vorhergesagten Hydrolasen oder Acyltransferasen, alpha/beta-Hydrolase-Superfamilie;
NCg12640, hypothetisches Protein (uncharakterisierter BCR);
NCgl2641, hypothetisches Protein, keine Annotation;
Tn*5531*, Transposon 5531 (*IS1207*). Das gefüllte Dreieck stellt die Stelle der Insertion von Tn5531 in NCg12640 dar.

### Abb. 3 Wachstumskurven von C. glutamicum-Wildtyp und Mutanten, die in CGXII-Medium in Gegenwart oder Abwesenheit von Ethionin angezogen wurden

Die Glukosekonzentration betrug 3 g l⁻¹; die D, L-Ethionin-Konzentrationen betrugen 3,5 g l⁻¹ oder 7,5 g l⁻¹ für den Wildtyp oder Mutantenstrang. Es muss beachtet werden, dass der Wildtyp nicht in Gegenwart von 7,5 g l⁻¹ D, L-Ethionin wächst.
Gefüllte Symbole: Ethionin vorhanden während der Kultivierung;
offene Symbole: Kultivierung ohne Ethionin;
A Wildtyp;
● mutanter Stamm.

### Abb. 4 Einfluss des NCg12640 Knock-outs auf die Methionin-abhängige Expression von cysK, metY und das Sulfatoperon

*C. glutamicum-*ATCC 14752 (Wildtyp; WT), der mutierte Stamm 14752::2640 und mutierte Stämme, die mit dem Plasmid-ständigen NCg12640 (::2640-cpl) komplementiert waren, und die Reporterplasmide pClik-P*cysK*, pClik-P*metY* oder pClik-Psulfat enthielten, wurden in CGXII (3 g l-1 Glucose)-Medium in Gegenwart oder Abwesenheit von Methionin (10 mM) angezogen. Die Promotor-Aktivität wurde mittels LacZ-Aktivitätsreporter-Assays bestimmt und in Form von Miller-Einheiten quantifiziert.
Dunkle Balken: kein Methionin im Wachstumsmedium;
leere Balken: L-Methionin im Wachstumsmedium.

### Abb. 5 SDS-PAGE für Proteine, die an putative Promotorregionen der C. glutamicum-Gene für Cystein-Synthase (cysK), O-Acetylhomoserin-Sulfhydrolase (metY) und Gene des putativen Sulfatoperons binden

1, TetR-ähnlicher Regulator McbR, der aus Rey et al. *(vide supra*) bekannt war;
2, Exopoly-Phosphatase (Abbauprodukt);
3, Exopoly-Phosphatase;
4, ATP-Phosphoribosyl-Transferase;
5, DNA-Polymerase I.

Mit Ausnahme der Exopoly-Phosphatase und der DNA-Polymerase I band keines der identifizierten Proteine an das *groES* Kontroll-Promotorfragment.

GAP-DH, Glyceraldehyd-3-phosphat-Dehydrogenase wurde als Markerprotein (37 kDa) verwendet;
M, Proteinmarker.

### Abb. 6 Extra- (A) und intrazelluläre (B) L-Methionin-Mengen in C. glutamicum-Wildtyp und im NCg12640 Knock-out Mutantenstamm (::2640) in Gegenwart oder Abwesenheit von Ethionin

Dunkle Balken: kein Ethionin während der Inkubation,
weiße Balken: 3,5 g l⁻¹ (Wildtyp) oder 7,5 g l⁻¹ (::2640) D, L-Ethionin.

**Tabelle 1: Zusammenfassung der vorstehend genannten Stämme und Plasmide.**

| **Stamm oder Plasmid** | **Phänotyp** | **Quelle oder Referenz** |
|---|---|---|
| *C. glutamicum-*Stämme | | |
| ATCC 13032 | Wildtyp | ATCC |
| ATCC 14752 | Wildtyp | (Simic et al., 2001) |
| 13032::2640 | Wildtyp mit *NCgl2640,* Km^{r} | erfindungsgemäß |
| 14752::2640 | Wildtype mit *NCgl2640,* Km^{r} | erfindungsgemäß |
| 14752*-*P*cysK* | ATCC 14752 mit Reporter-plasmid pClik-P*cysK*, Cm^{r} | erfindungsgemäß |
| CG::2640-P*cysK* | 14752::2640 mit Reporterplasmid pClik-P*cysK*, Km^{r} Cm^{r} | erfindungsgemäß |
| CG::2640-P*cysK*-cpl | 14752::2640 mit Reporterplasmid pClik-P*cysK*-cpl, Km^{r} Cm^{r} | erfindungsgemäß |
| 14752-P*metY* | ATCC 14752 mit Reporterplasmid pClik-P*metY*, Cm^{r} | erfindungsgemäß |
| CG::2640-P*metY* | 14752::2640 mit Reporterplasmid pClik-P*metY*, Km^{r} Cm^{r} | erfindungsgemäß |
| CG::2640-P*metY*-cpl | 14752::2640 mit Reporterplasmid pClik-P*metY-*cpl, Km^{r} Cm^{r} | erfindungsgemäß |
| 14752-Psulfate | ATCC 14752 mit Reporterplasmid pClik-Psulfate, Cm^{r} | erfindungsgemäß |
| CG::2640-Psulfate | 14752::2640 mit Reporterplasmid pClik-Psulfate, Km^{r} Cm^{r} | erfindungsgemäß |
| CG::2640-Psulfate-cpl | 14752::2640 mit Reporterplasmid | erfindungsgemäß |
| | pClik-Psulfate-cpl, Km^{r} Cm^{r} | |
| *E. coli*-Stämme | | |
| DH5α | F⁻ *endA1*, *hsdR17*(rk*⁻*mk⁺) *supE44*, *thi-l λ⁻ recA1 gyrA96 relA1 φ80ΔlacAm15* | (Hanahan, 1983) |
| ET12567 | *dam dcm hsd,* restriktionsdefizient | (MacNeil et al., 1992) |
| Plasmide | | |
| pUC18 | Ap^{r}, *lacZ* | Stratagene |
| pCGL0040 | Donor von Tn*5531* (IS*1207* Km^{r}); Ap^{r}, *oriV_{E.c}.* | (Ankri et al., 1996b) |
| pMT1 | *E.coli - C. glutamicum* Shuttle-Vektor, Ap^{r} Km^{r} | (Follettie et al., 1993; Lee and Sinskey, 1994) |
| öpClik-SacB | Vektor zum allelischen Austausch durch homologe Rekombination; nicht-replikativ in *C. glutamicum* Km^{r}, SacB | (Hwang et al., 1999) |
| pSdel-NCgl2640 | pClikSacB-basierender allelischer Austauschvektor zur chromosomalen Deletion von NCgl2640 | erfindungsgemäß |
| pClik-PcysK | *cysK*-Promotersonden-Vektor *lacZ*-Fusion, Cm^{r} | erfindungsgemäß |
| pClik-PcysK-cpl | pClik-P*cysK* mit *NCgl2640,* Cm^{r} | erfindungsgemäß |
| pClik-PmetY | *metY-* Promotersonden-Vektor *lac*Z-Fusion, Cm^{r} | erfindungsgemäß |
| pClik-PmetY-cpl | pClik-P*metY* mit *NCgl2640,* Cm^{r} | erfindungsgemäß |
| pClik-Psulfate | Sulfate-operon- Promotersonden-Vektor *lac*Z-Fusion, Cm^{r} | erfindungsgemäß |
| pClik-Psulfate-cpl | pClik-Psulfate mit *NCgl2640,* Cm^{r} | erfindungsgemäß |

### Literaturangaben in Tabelle 1:

Ankri, S., Serebrijski, I., Reyes, O., and Leblon, G. (1996b) Mutations in the Corynebacterium glutamicum proline biosynthetic pathway: a natural bypass of th proA step. J Bacteriol 178, 4412-4419.
Follettie, M. T., Peoples, O. P., Agoropoulou, C., and Sinskey, A. J. (1993) Gene structure and expression of the Corynebacterium flavum N13 ask-asd operon. J Bacteriol 175, 4096-4103.
Hanahan, D. (1983) Studies on transformation of Escherichia coli with plasmids. J Mol Biol 166, 557-580.
Hwang, B.-J., Kim, Y., Kim, H.-B., Hwang, H.-J., Kim, J.-H., and Lee, H. S. (1999) Analysis of Corynebacterium glutamicum methionine biosynthetic pathway: isolation and analysis of metB encoding cystathionine γ-synthase. Mol Cells 9, 300-308.
Lee, H.-S., and Sinskey, A. J. (1994) Molecular characterization of AceB, a gene encoding malate synthase in Corynebacterium glutamicum. J Microbiol Biotechnol 4, 256-263.
MacNeil, D. J., Occi, J. L., Gewain, K. M., MacNeil, T., Gibbons, P. H., Ruby, C. L., and Danis, S. J. (1992) Complex organization of the Streptomyces avermitilis genes encoding the avermectin polyketide synthase. Gene 115, 119-125.
Simic, P., Sahm, H., and Eggeling, L. (2001) L-threonine export: use of peptides to identify a new translocator from Corynebacterium glutamicum. J Bacteriol 183, 5317-5324.

## Patentansprüche

1. Corynebacterium zur Herstellung von schwefelhaltigen Verbindungen,
**dadurch gekennzeichnet, dass** im Vergleich zum jeweiligen Wildtyp des Mikroorganismus der Gehalt des Proteins, das durch eine Nukleinsäure kodiert ist, die eine Sequenz aufweist, die zur Sequenz SEQ ID NO: 1 mindestens zu 95% identisch ist, durch Disruption und/oder Deletion der entsprechenden genomischen Nukleinsäuresequenz(en) erniedrigt ist.

2. Corynebacterium gemäß den Anspruch 1, **dadurch gekennzeichnet, dass** die Nukleinsäure für ein Protein mit der Aminosäuresequenz SEQ ID NO: 2 kodiert.

3. Corynebacterium nach den Ansprüchen 1 und 2,
**dadurch gekennzeichnet, dass** die schwefelhaltigen Verbindungen L-Methionin, L-Cystein, L-Homocystein, L-Cystathionin, S-Adenosyl-L-Methionin, Glutathion, Biotin, Thiamin und/oder Liponsäure, umfassen.

4. Corynebacterium gemäß den Ansprüchen 1 bis 3,
**dadurch gekennzeichnet, dass** der Gehalt des Proteins, für die die Nukleinsäure kodiert, ausgewählt ist aus der Gruppe bestehend aus:
der für Methionin-Synthase kodierenden Nukleinsäure metH,
der für Aspartatkinase kodierenden Nukleinsäure lysC,
der für Glycerinaldehyd-3-Phosphat-Dehydrogenase kodierenden Nukleinsäure gap,
der für 3-Phosphoglycerat-Kinase kodierenden Nukleinsäure pgk,
der für Pyruvat-Carboxylase kodierenden Nukleinsäure pyc,
der für Triosephosphat-Isomerase kodierenden Nukleinsäure tpi,
der für Homoserin-O-Acetyltransferase kodierenden Nukleinsäure metA,
der für Cystathionin-gamma-Synthase kodierenden Nukleinsäure metB,
der für Cystathionin-gamma-Lyase kodierenden Nukleinsäure metC,
der für Serin-Hydroxymethyltransferase kodierenden Nukleinsäure glyA,
der für O-Acetylhomoserin-Sulfhydrylase kodierenden Nukleinsäure metY,
der für Methionin-Synthase kodierenden Nukleinsäure metH,
der für Phosphoserin-Aminotransferase kodierenden Nukleinsäure serC,
der für Phosphoserin-Phosphatase kodierenden Nukleinsäure serB,
der für Serinacetyl-Transferase kodierenden Nukleinsäure cysE,
der für Homoserin-Dehydrogenase kodierenden Nukleinsäure hom,
der für Methionin-Synthase kodierenden Nukleinsäure metH,
der für Methionin-Synthase kodierenden Nukleinsäure metE,
einer für Cystein-Synthase kodierenden Nukleinsäure,
einer für Sulfit-Reduktase kodierenden Nukleinsäure,
der für Phosphoadenosin-Phosphosulfat-Reduktase kodierenden Nukleinsäure cysH,
einer für Sulfatadenylyltransferase-Subunit 1 kodierenden Nukleinsäure,
einer für CysN-Sulfatadenylyltransferase-Subunit 2 kodierenden Nukleinsäure,
einer für Ferredoxin-NADP-Reduktase kodierenden Nukleinsäure,
einer für Ferredoxin kodierenden Nukleinsäure,
einer für Glucose-6-Phosphat-Dehydrogenase kodierenden Nukleinsäure,
und/oder
einer für Fructose-1-6-Bisphosphatase kodierenden Nukleinsäure,
im Vergleich zum jeweiligen Wildtyp erhöht ist (sind).

5. Corynebacterium nach den Ansprüchen 1 bis⁴,
**dadurch gekennzeichnet, dass** der Gehalt und/oder die Aktivität des Proteins, für die die Nukleinsäure kodiert, ausgewählt aus der Gruppe, bestehend aus
der für Homoserine-Kinase kodierenden Nukleinsäure thrB,
der für Threonin-Dehydratase kodierenden Nukleinsäure ilvA,
der für Threonin-Synthase kodierenden Nukleinsäure thrC,
der für Meso-Diaminopimelat-D-Dehydrogenase kodierenden Nukleinsäure ddh,
der für Phosphoenolpyruvat-Carboxykinase kodierenden Nukleinsäure pck,
der für Glucose-6-Phosphat-6-Isomerase kodierenden Nukleinsäure pgi, der für Pyruvat-Oxidase kodierenden Nukleinsäure poxB,
der für Dihydrodipicolinat-Synthase kodierenden Nukleinsäure dapA, der für Dihydrodipicolinat-Reduktase kodierenden Nukleinsäure dapB; der für Diaminopicolinat-Decarboxylase kodierenden Nukleinsäure lysA; einer für Glycosyltransferase kodierenden Nukleinsäure, und/oder einer für Laktatdehydrogenase kodierenden Nukleinsäure,
im Vergleich zum jeweiligen Wildtyp verringert ist (sind).

6. Verfahren zur Herstellung von schwefelhaltigen Verbindungen in Mikroorganismen, umfassend das Kultivieren eines Corynebacteriums nach einem der Ansprüche 1 bis 5.

7. Verfahren nach Anspruch 6**,**
**dadurch gekennzeichnet, dass** es sich um ein Fermentationsverfahren handelt, bei dem die schwefelhaltigen Verbindungen im Medium und/oder in den Zellen der Mikroorganismen angereichert und daraus isoliert werden.

8. Verfahren nach Anspruch 6 oder 7,
**dadurch gekennzeichnet, dass** die schwefelhaltigen Verbindungen L-Methionin, L-Cystein, L-Homocystein, L-Cystathionin, S-Adenosyl-L-Methionin, Glutathion, Biotin, Thiamin und/oder Liponsäure umfassen.

9. Verfahren nach den Ansprüchen 6 bis 8,
**dadurch gekennzeichnet, dass** der Mikroorganismus *Corynebacterium glutamicum* ist.

10. Verfahren nach einem der Ansprüche 6 bis 9,
**dadurch gekennzeichnet, dass** im Vergleich zum Wildtyp des Mikroorganismus zusätzlich der Gehalt und/oder die Aktivität von Proteinen erniedrigt wird, die durch Nukleinsäuren kodiert sind, die identisch zu Nukleinsäuren mit der Sequenz SEQ ID No 3 sind.

## Claims

1. Corynebacterium for producing sulphur-containing compounds,
**characterized in that,** in comparison with the respective wild type of the microorganism, the content of the protein which is encoded by a nucleic acid which includes a sequence with at least 95% identity to the sequence SEQ ID NO: 1 is reduced by disrupting and/or deleting the respective genomic nucleic acid sequence(s).

2. Corynebacterium according to Claim 1, **characterized in that** the nucleic acid codes for a protein with the amino acid sequence SEQ ID NO: 2.

3. Corynebacterium according to Claims 1 and 2, **characterized in that** the sulphur-containing compounds comprise L-methionine, L-cysteine, L-homocysteine, L-cystathionine, S-adenosyl-L-methionine, glutathione, biotin, thiamine and/or lipoic acid.

4. Corynebacterium according to Claims 1 to 3, **characterized in that** the content of the protein for which the nucleic acid codes is selected from the group consisting of:
the nucleic acid metH, which codes for methionine synthase,
the nucleic acid lysC, which codes for aspartate kinase,
the nucleic acid gap, which codes for glyceraldehydes-3-phosphate dehydrogenase,
the nucleic acid pgk, which codes for 3-phospho-glycerate kinase,
the nucleic acid pyc, which codes for pyruvate carboxylase,
the nucleic acid tpi, which codes for triosephosphate isomerase,
the nucleic acid metA, which codes for homoserine O-acetyltransferase,
the nucleic acid metB, which codes for cystathionine gamma-synthase,
the nucleic acid metC, which codes for cystathionine gamma-lyase,
the nucleic acid glyA, which codes for serine hydroxymethyltransferase,
the nucleic acid metY, which codes for O-acetyl-homoserine sulphhydrylase,
the nucleic acid metH, which codes for methionine synthase,
the nucleic acid serC, which codes for phosphoserine aminotransferase,
the nucleic acid serB, which codes for phosphoserine phosphatase,
the nucleic acid cysE, which codes for serine acetyltransferase,
the nucleic acid hom, which codes for homoserine dehydrogenase,
the nucleic acid metH, which codes for methionine synthase,
the nucleic acid metE, which codes for methionine synthase,
a nucleic acid which codes for cysteine synthase,
a nucleic acid which codes for sulphite reductase,
the nucleic acid cysH, which codes for phosphoadenosine phosphosulphate reductase,
a nucleic acid which codes for sulphate adenylyltransferase subunit 1,
a nucleic acid which codes for CysN-sulphate adenylyltransferase subunit 2,
a nucleic acid which codes for ferredoxin-NADP reductase,
a nucleic acid which codes for ferredoxin,
a nucleic acid which codes for glucose-6-phosphate dehydrogenase, and/or
a nucleic acid which codes for fructose-1,6-bis-phosphatase,
is (are) enhanced in comparison with the respective wild type.

5. Corynebacterium according to Claims 1 to 4,
**characterized in that** the content and/or the activity of the protein for which the nucleic acid codes, selected from the group consisting of
the nucleic acid thrB, which codes for homoserine kinase,
the nucleic acid ilvA, which codes for threonine dehydratase,
the nucleic acid thrC, which codes for threonine synthase,
the nucleic acid ddh, which codes for mesodiaminopimelate D-dehydrogenase,
the nucleic acid pck, which codes for phosphoenolpyruvate carboxykinase,
the nucleic acid pgi, which codes for glucose-6-phosphate-6-isomerase,
the nucleic acid poxB, which codes for pyruvate oxidase,
the nucleic acid dapA, which codes for dihydrodipicolinate synthase,
the nucleic acid dapB, which codes for dihydrodipicolinate reductase,
the nucleic acid lysA, which codes for diaminopicolinate decarboxylase,
a nucleic acid which codes for glycosyltransferase and/or
a nucleic acid which codes for lactate dehydrogenase
is (are) reduced in comparison with the respective wild type.

6. Process for the production of sulphur-containing compounds in microorganisms, comprising the culturing of a Corynebacterium according to any of Claims 1 to 5.

7. Process according to Claim 6,
**characterized in that** the fermentation process is one in which the sulphur-containing compounds are accumulated in the medium and/or in the cells of the microorganisms and isolated therefrom.

8. Process according to Claim 6 or 7,
**characterized in that** the sulphur-containing compounds comprise L-methionine, L-cysteine, L-homocysteine, L-cystathionine, S-adenosyl-L-methionine, glutathione, biotin, thiamine and/or lipoic acid.

9. Process according to Claims 6 to 8,
**characterized in that** the microorganism is *Corynebacterium glutamicum.*

10. Process according to any of Claims 6 to 9,
**characterized in that** additionally the content and/or the activity of proteins which are encoded by nucleic acids which are identical to nucleic acids with the sequence SEQ ID No. 3 is reduced in comparison with the wild type of the microorganism.

## Revendications

1. Corynebacterium pour la préparation de composés sulfurés, **caractérisé en ce que**, par comparaison avec le type sauvage considéré du microorganisme, la teneur en la protéine qui est codée par un acide nucléique qui présente une séquence ayant une identité d'au moins 95 % avec la séquence SEQ ID NO:1, est abaissée par cassure et/ou délétion de la ou des séquences d'acide nucléique génomiques correspondantes.

2. Corynebacterium selon la revendication 1, **caractérisé en ce que** l'acide nucléique code pour une protéine ayant la séquence d'acides aminés SEQ ID NO:2.

3. Corynebacterium selon les revendications 1 et 2, **caractérisé en ce que** les composés sulfurés comprennent la L-méthionine, la L-cystéine, la L-homocystéine, la L-cystathionine, la S-adénosyl-L-méthionine, le glutathion, la biotine, la thiamine et/ou l'acide lipoïque.

4. Corynebacterium selon les revendications 1 à 3, **caractérisé en ce que** la teneur en la protéine pour laquelle code l'acide nucléique, choisi dans le groupe consistant en :
l'acide nucléique metH codant pour la méthionine-synthase,
l'acide nucléique lysC codant pour l'aspartatekinase,
l'acide nucléique gap codant pour la glycérinaldéhyde-3-phosphate-déshydrogénase,
l'acide nucléique pgk codant pour la 3-phosphoglycérate-kinase,
l'acide nucléique pyc codant pour la pyruvate-carboxylase,
l'acide nucléique tpi codant pour la triosephosphate-isomérase,
l'acide nucléique metA codant pour l'homosérine-O-acétyltransférase,
l'acide nucléique metB codant pour la cystathionine-gamma-synthase,
l'acide nucléique metC codant pour la cystathionine-gamma-lyase,
l'acide nucléique glyA codant pour la sérine-hydroxyméthyltransférase,
l'acide nucléique metY codant pour l'O-acétylhomosérine-sulfhydrylase,
l'acide nucléique metH codant pour la méthionine-synthase,
l'acide nucléique serC codant pour la phosphosérine-aminotransférase,
l'acide nucléique serB codant pour la phosphosérine-phosphatase,
l'acide nucléique cysE codant pour la sérinacétyltransférase,
l'acide nucléique hom codant pour l'homosérine-déshydrogénase,
l'acide nucléique metH codant pour la méthionine-synthase,
l'acide nucléique metE codant pour la méthionine-synthase,
un acide nucléique codant pour la cystéine-synthase,
un acide nucléique codant pour la sulfite-réductase,
l'acide nucléique cysH codant pour la phosphoadénosine-phosphosulfate-réductase,
un acide nucléique codant pour la sous-unité 1 de la sulfate-adénylyltransférase,
un acide nucléique codant pour la sous-unité 2 de la CysN-sulfate-adénylyltransférase,
un acide nucléique codant pour la ferredoxine-NADP-réductase,
un acide nucléique codant pour la ferredoxine,
un acide nucléique codant pour la glucose-6-phosphate-déshydrogénase, et/ou
un acide nucléique codant pour la fructose-1-6-bisphosphatase,
est augmentée par comparaison avec celle du type sauvage correspondant.

5. Corynebacterium selon les revendications 1 à 4, **caractérisé en ce que** la teneur et/ou l'activité de la protéine pour laquelle code l'acide nucléique choisi dans le groupe consistant en
l'acide nucléique thrB codant pour l'homosérine-kinase,
l'acide nucléique ilvA codant pour la thréonine-déshydratase,
l'acide nucléique thrC codant pour la thréonine-synthase,
l'acide nucléique ddh codant pour la mésodiaminopimélate-D-déshydrogénase,
l'acide nucléique pck codant pour la phosphoénolpyruvate-carboxykinase,
l'acide nucléique pgi codant pour la glucose-6-phosphoate-6-isomérase,
l'acide nucléique poxB codant pour la pyruvate-oxydase,
l'acide nucléique dapA codant pour la dihydrodipicolinate-synthase,
l'acide nucléique dapB codant pour la dihydrodipicolinate-réductase ;
l'acide nucléique lysA codant pour la diaminopicolinate-décarboxylase,
un acide nucléique codant pour la glycosyltransférase, et/ou
un acide nucléique codant pour la lactate-déshydrogénase,
est/sont abaissée(s) par rapport au type sauvage correspondant

6. Procédé de préparation de composés sulfurés dans des microorganismes, comprenant la culture d'un Corynebacterium selon l'une des revendications 1 à 5.

7. Procédé selon la revendication 6, **caractérisé en ce qu'**il s'agit d'un procédé de fermentation dans lequel les composés sulfurés sont enrichis dans le milieu et/ou dans les cellules des microorganismes, et en sont isolés.

8. Procédé selon la revendication 6 ou 7, **caractérisé en ce que** les composés sulfurés comprennent la L-méthionine, la L-cystéine, la L-homocystéine, la L-cystathionine, la S-adénosyl-L-méthionine, le glutathion, la biotine, la thiamine et/ou l'acide lipoïque.

9. Procédé selon les revendications 6 à 8, **caractérisé en ce que** le microorganisme est *Corynebacterium glutamicum.*

10. Procédé selon l'une des revendications 6 à 9, **caractérisé en ce que**, par comparaison avec le type sauvage du microorganisme, on a en plus un abaissement de la teneur et/ou de l'activité de protéines qui sont codées par des acides nucléiques qui sont identiques aux acides nucléiques ayant la séquence SEQ ID NO:3.


### SEQUENCE LISTING

<110> BASF AG
<120> Mikroorganisms for the production of sulfur-containing compounds
<130> B 7673
<140> DE 1.0 2004 035 052.3
   <141> 2004-07-20
<160> 11
<170> PatentIn version 3.3
<210> 1
   <211> 1131
   <212> DNA
   <213> Corynebacterium glutamicum
<300>
   <308> GenBank/NCgl2640
   <309> 2002-03-20
   <313> (1)..(1131)
<400> 1
   atgggcattg agtttaagcg ttcaccgcga cccaccctgg gcgttgagtg ggaaattgca 60
   cttgttgatc cagaaacacg tgatctagcc ccgcgcgctg cagaaatact agagattgtg 120
   gccaagaacc accctgaggt gcacctcgag cgcgaattcc tccaaaacac cgtggagctt 180
   gtcaccggag tgtgcgacac cgtccccgaa gcggtggcag agctttccca cgatctagat 240
   gcgctgaaag aagcagcgga ttctctcggg cttcggttgt ggacctctgg atcccaccca 300
   ttttcggatt tccgcgaaaa cccagtatct gaaaaaggct cctacgacga gatcatcgcg 360
   cgcacccaat actggggaaa ccagatgttg atttggggca ttcacgtcca cgtgggcatc 420
   agccatgaag atcgcgtgtg gccgatcatc aatgcgctgc tgacaaatta cccacatctg 480
   ttggcacttt ctgcaagctc tccagcatgg gacggacttg ataccggtta tgcctccaac 540
   cggacgatgc tctaccaaca gctgcctaca gccggactgc cataccaatt ccaaagctgg 600
   gatgaatggt gcagctacat ggcggatcaa gataaatccg gtgtcatcaa ccacaccgga 660
   tccatgcact ttgatatccg ccccgcatcc aaatggggaa ccatcgaagt ccgcgtggcc 720
   gattctacct ccaacctgcg ggaactgtct gccatcgtgg cgttgaccca ctgtctcgtg 780
   gtgcactacg accgcatgat cgacgctggc gaagagcttc cctccctgca acaatggcac 840
   gtttcggaaa ataaatggcg cgcggctagg tatggtctgg atgccgaaat catcatttcc 900
   agagacaccg atgaagcgat ggttcaagac gaactccgcc gactagtagc gcaattgatg 960
   cctctagcca acgaactcgg ctgcgctcgt gagcttgaac ttgtgttgga aatcctggaa 1020
   cgtggtggtg gatacgaacg ccaacgcaga gtgtttaaag aaactggcag ttggaaagct 1080
   gcagttgatt tagcctgcga cgaactcaac gacctcaaag cactggacta a 1131
<210> 2
   <211> 376
   <212> PRT
   <213> Corynebacterium glutamicum
<300>
   <308> GenBank/NP_601931
   <309> 2002-03-20
   <313> (1) .. (376)
<400> 2
   Met Gly Ile Glu Phe Lys Arg Ser Pro Arg Pro Thr Leu Gly Val Glu
   1 5 10 15
   Trp Glu Ile Ala Leu Val Asp Pro Glu Thr Arg Asp Leu Ala Pro Arg
   20 25 30
   Ala Ala Glu Ile Leu Glu Ile Val Ala Lys Asn His Pro Glu Val His
   35 40 45
   Leu Glu Arg Glu Phe Leu Gln Asn Thr Val Glu Leu Val Thr Gly Val
   50 55 60
   Cys Asp Thr Val Pro Glu Ala Val Ala Glu Leu Ser His Asp Leu Asp
   65 70 75 80
   Ala Leu Lys Glu Ala Ala Asp Ser Leu Gly Leu Arg Leu Trp Thr Ser
   85 90 95
   Gly Ser His Pro Phe Ser Asp Phe Arg Glu Asn Pro Val Ser Glu Lys
   100 105 110
   Gly Ser Tyr Asp Glu Ile Ile Ala Arg Thr Gln Tyr Trp Gly Asn Gln
   115 120 125
   Met Leu Ile Trp Gly Ile His Val His Val Gly Ile Ser His Glu Asp
   130 135 140
   Arg Val Trp Pro Ile Ile Asn Ala Leu Leu Thr Asn Tyr Pro His Leu
   145 150 155 160
   Leu Ala Leu Ser Ala Ser Ser Pro Ala Trp Asp Gly Leu Asp Thr Gly
   165 170 175
   Tyr Ala Ser Asn Arg Thr Met Leu Tyr Gln Gln Leu Pro Thr Ala Gly
   180 185 190
   Leu Pro Tyr Gln Phe Gln Ser Trp Asp Glu Trp Cys Ser Tyr Met Ala
               195                 200                 205
   Asp Gln Asp Lys Ser Gly Val Ile Asn His Thr Gly Ser Met His Phe
       210                 215                 220
   Asp Ile Arg Pro Ala Ser Lys Trp Gly Thr Ile Glu Val Arg Val Ala
   225                 230                 235                 240
   Asp Ser Thr Ser Asn Leu Arg Glu Leu Ser Ala Ile Val Ala Leu Thr
                   245                 250                 255
   His Cys Leu Val Val His Tyr Asp Arg Met Ile Asp Ala Gly Glu Glu
               260                 265                 270
   Leu Pro Ser Leu Gln Gln Trp His Val Ser Glu Asn Lys Trp Arg Ala
           275                 280                 285
   Ala Arg Tyr Gly Leu Asp Ala Glu Ile Ile Ile Ser Arg Asp Thr Asp
       290                 295                 300
   Glu Ala Met Val Gln Asp Glu Leu Arg Arg Leu Val Ala Gln Leu Met
   305                 310                 315                 320
   Pro Leu Ala Asn Glu Leu Gly Cys Ala Arg Glu Leu Glu Leu Val Leu
                   325                 330                 335
   Glu Ile Leu Glu Arg Gly Gly Gly Tyr Glu Arg Gln Arg Arg Val Phe
               340                 345                 350
   Lys Glu Thr Gly Ser Trp Lys Ala Ala Val Asp Leu Ala Cys Asp Glu
           355                 360                 365
   Leu Asn Asp Leu Lys Ala Leu Asp
       370                 375
<210> 3
   <211> 642
   <212> DNA
   <213> Corynebacterium glutamicum
<300>
   <308> GenBank/AY217661
   <309> 2003-07-30
   <313> (1) .. (692)
<400> 3
   gtggctgcta gcgcttcagg caagagtaaa acaagtgccg gggcaaaccg tcgtcgcaat 60
   cgaccaagcc cccgacagcg tctcctcgat agcgcaacca accttttcac cacagaaggt 120
   attcgcgtca tcggtattga tcgtatcctc cgtgaagctg acgtggcgaa ggcgagcctc 180
   tattcccttt tcggatcgaa ggacgccttg gttattgcat acctggagaa cctcgatcag 240
   ctgtggcgtg aagcgtggcg tgagcgcacc gtcggtatga aggatccgga agataaaatc 300
   atcgcgttct ttgatcagtg cattgaggaa gaaccagaaa aagatttccg cggctcgcac 360
   tttcagaatg cggctagtga gtaccctcgc cccgaaactg atagcgaaaa gggcattgtt 420
   gcagcagtgt tagagcaccg cgagtggtgt cataagactc tgactgattt gctcactgag 480
   aagaacggct acccaggcac cacccaggcg aatcagctgt tggtgttcct tgatggtgga 540
   cttgctggat ctcgattggt ccacaacatc agtcctcttg agacggctcg cgatttggct 600
   cggcagttgt tgtcggctcc acctgcggac tactcaattt ag 642
<210> 4
   <211> 213
   <212> PRT
   <213> Corynebacterium glutamicum
<300>
   <308> GenBank/NP_602128
   <309> 2002-03-20
   <313> (1) .. (213)
<400> 4
   Met Ala Ala Ser Ala Ser Gly Lys Ser Lys Thr Ser Ala Gly Ala Asn
   1 5 10 15
   Arg Arg Arg Asn Arg Pro Ser Pro Arg Gln Arg Leu Leu Asp Ser Ala
   20 25 30
   Thr Asn Leu Phe Thr Thr Glu Gly Ile Arg Val Ile Gly Ile Asp Arg
   35 40 45
   Ile Leu Arg Glu Ala Asp Val Ala Lys Ala Ser Leu Tyr Ser Leu Phe
   50 55 60
   Gly Ser Lys Asp Ala Leu Val Ile Ala Tyr Leu Glu Asn Leu Asp Gln
   65 70 75 80
   Leu Trp Arg Glu Ala Trp Arg Glu Arg Thr Val Gly Met Lys Asp Pro
   85 90 95
   Glu Asp Lys Ile Ile Ala Phe Phe Asp Gln Cys Ile Glu Glu Glu Pro
   100 105 110
   Glu Lys Asp Phe Arg Gly Ser His Phe Gln Asn Ala Ala Ser Glu Tyr
   115 120 125
   Pro Arg Pro Glu Thr Asp Ser Glu Lys Gly Ile Val Ala Ala Val Leu
   130 135 140
   Glu His Arg Glu Trp Cys His Lys Thr Leu Thr Asp Leu Leu Thr Glu
   145 150 155 160
   Lys Asn Gly Tyr Pro Gly Thr Thr Gln Ala Asn Gln Leu Leu Val Phe
   165 170 175
   Leu Asp Gly Gly Leu Ala Gly Ser Arg Leu Val His Asn Ile Ser Pro
   180 185 190
   Leu Glu Thr Ala Arg Asp Leu Ala Arg Gln Leu Leu Ser Ala Pro Pro
   195 200 205
   Ala Asp Tyr Ser Ile
   210
<210> 5
   <211> 35
   <212> DNA
   <213> Artificial
<220>
   <223> Primer 2640-fwd-BglII
<400> 5
   ccgctgctgc tggtggcgct agatctgcta acggc 35
<210> 6
   <211> 35
   <212> DNA
   <213> Artificial
<220>
   <223> Primer 2640-rev-BglII
<400> 6
   atgtgttggg agatctctta agttatttag tccag 35
<210> 7
   <211> 23
   <212> DNA
   <213> Artificial
<220>
   <223> oligonucleotide Tn5531-Eco
<400> 7
   cgggtctaca ccgctagccc agg 23
<210> 8
   <211> 29
   <212> DNA
   <213> Artificial
<220>
   <223> Primer 2640-SacB1
<400> 8
   gagagggccc atcagcagaa cctggaacc 29
<210> 9
   <211> 19
   <212> DNA
   <213> Artificial
<220>
   <223> Primer 2640-SacB2
<400> 9
   gatccagagg tccacaacc 19
<210> 10
   <211> 20
   <212> DNA
   <213> Artificial
<220>
   <223> Primer 2640-SacB3
<400> 10
   gatggttcaa gacgaactcc 20
<210> 11
   <211> 30
   <212> DNA
   <213> Artificial
<220>
   <223> Primer 2640-SacB4
<400> 11
   gagagtcgac cagaatcaat tccagccttc 30